# EUROPEAN PATENT SPECIFICATION

(11) **EP 0 550 550 B1**
(45) Date of publication and mention of the grant of the patent: **16.07.2003**
(21) Application number: 91916973.0
(22) Date of filing: 23.09.1991
(51) Int. Cl.: C07D 471/08, A61K 31/495, C07D 519/00

(54) **3,9-DIAZABICYCLO (3.3.1) NONAN-7-YL DERIVATIVES, PROCESS AND INTERMEDIATES FOR THEIR PREPARATION AND PHARMACEUTICAL COMPOSITIONS CONTAINING THEM**
3,9-DIAZABICYCLO-[3.3.1]-NONANYLDERIVATE, VERFAHREN UND INTERMEDIATE FÜR IHRE DARSTELLUNG UND SIE ENTHALTENDE PHARMAZEUTISCHE ZUSAMMENSETZUNGEN
DERIVES DE 3,9-DIAZABICYCLO (3.3.1) NONAN-7-YL, PROCEDE ET INTERMEDIAIRES DE PREPARATION ET COMPOSITIONS PHARMACEUTIQUES LES CONTENANT

(30) Priority: 26.09.1990 GB 9020927
(43) Date of publication of application: 14.07.1993
(73) Proprietor: Beecham Group p.l.c., Brentford, Middlesex TW8 9GS (GB)
(72) Inventor: KING, F. D., c/oGlaxoSmithKline,, Essex CM19 5AD (GB); GREGORY, Julian, Anthony c/oGlaxoSmithKline,, Harlow, Essex, CM19 5AW (GB)
(74) Representative: Waters, David Martin
(86) International application number: GB9101629
(87) International publication number: WO92005174

(56) References cited:
- EP-A- 0 200 444
- GB-A- 2 193 633
- US-A- 4 920 219
- US-A- 4 920 227

## Description

This invention relates to compounds having pharmacological activity, to a process and intermediates for their preparation, and to their use as pharmaceuticals.

EP-A-158265, EP-A-200444, EP-A-247266, EP-A-235878, EP-A-254584, EP-A-255297, EP-A-289170, EP-A-315390 and PCT/GB91/00636 (Beecham Group p.l.c.), EP-A-158532 (A.H. Robins Company, Inc.), EP-A-67770 (Merrell Toraude et Compagnie), GB 2125398A and GB 2145416A (Sandoz Limited), EP-A-322016 and EP-A-436245 (Duphar international Research B.V.), EP-A-307172 (Eli Lilly and Company), EP-A-323077, EP-A-306148, GB 2208385A and WO91/05738 (John Wyeth and Brother Limited), EP-A-234872 (Adria Laboratories Inc.), EP-A-294292 (Adir et Compagnie), EP-A-339950 (Rorer International (overseas), Inc.), EP-A-309423 (Instituto de Angeli S.p.A.), EP-A-313393 and EP-A-407137 (Yoshitomi Pharmaceutical industries Limited), EP-A-328200 and EP-A-337547 (Merck Sharp and Dohme Limited), EP-A-329932 (Merrell Dow Pharmaceuticals Inc.), WO 90/06039 and WO 91/04738 (Rorer International (Overseas), Inc.), EP-A-378111 (Zambon Group S.p.A.), EP-A-430190 (Syntex (U.S.A.).Inc.) and USA Patents 4920219 and 4920227 (Rorer Pharmaceutical Corp.) disclose classes of compounds which have a saturated azabicyclic moiety, such as tropanyl, granatyl or quinuclidinyl, and are 5-HT₃ receptor antagonists.

A class of compounds has now been discovered in which the saturated azabicyclic moiety is endo-3,9-diazabicyclo[3.3.1]nonan-7-yl. These compounds have 5-HT₃ receptor antagonist activity.

The present invention provides a compound of formula (IA), or a pharmaceutically acceptable salt thereof: 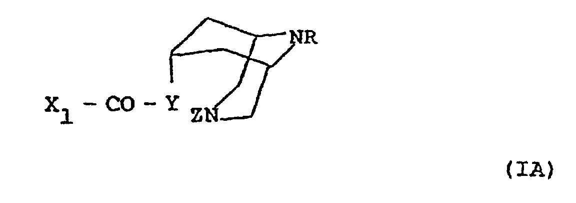 wherein
- Z: is C₁₋₆ alkyl, phenyl or phenyl C₁₋₄ alkyl wherein a phenyl moiety is optionally substituted by one or more of halo, C₁₋₆ alkoxy or C₁₋₆ alkyl;
- R: is methyl;
- Y: is NH or O (or is joined to R₁₀ as defined below); and
- X₁: is a group of formula (a), (b), (c), (d), (e), (f), or (g): 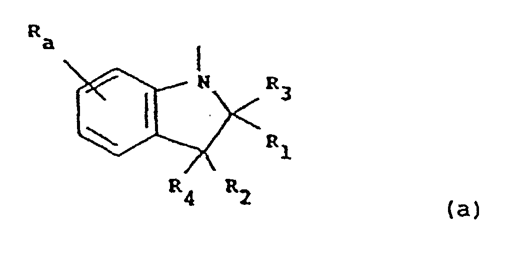 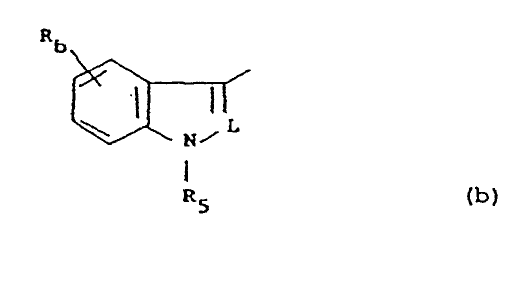 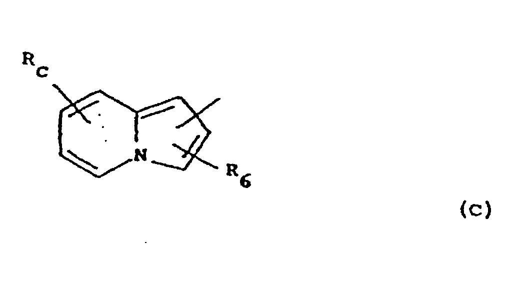 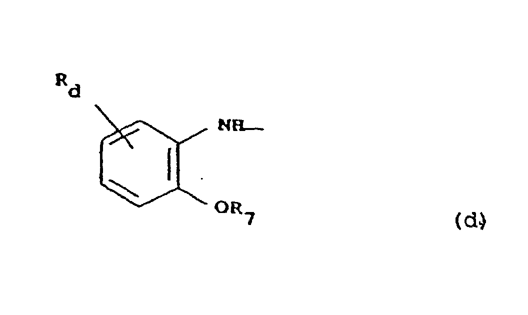 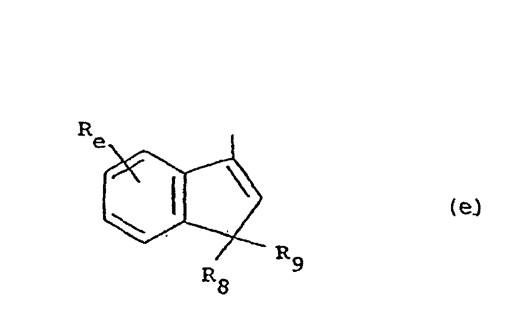 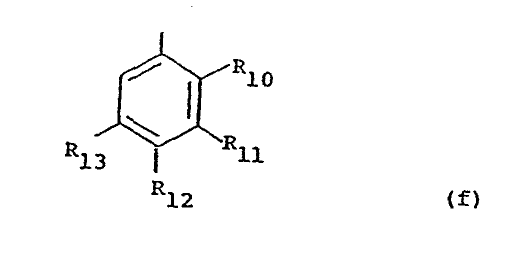 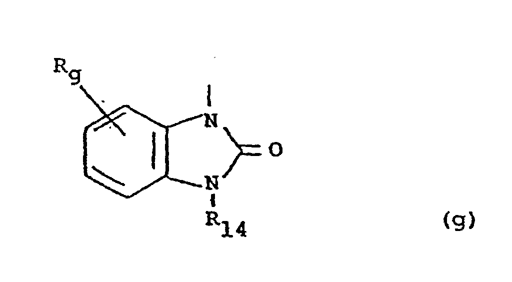
wherein
- Rₐ to Rₑ and R_{g}: are selected from hydrogen, halogen or hydroxy;
- R₁: is hydrogen and R₂ is hydrogen or C₁₋₄ alkyl; or
- R₁ and R₂: together are a bond;
- R₃ to R₇: are independently hydrogen or C₁₋₆ alkyl; and
- R₄: together with R₂ may be C₂₋₇ polymethylene or C₂₋₆ polymethylene interrupted by an -O- linkage when R₁ is hydrogen;
- R₈ and R₉: are independently selected from hydrogen or C₁₋₆ alkyl or R₈ and R₉ together are C₂₋₆ polymethylene or C₂₋₅ polymethylene interrupted by an -O- linkage;
- either R₁₀: is hydrogen, C₁₋₆ alkoxy, C₃₋₈ cycloalkyloxy or C₃₋₈ cycloalkyl C₁₋₄ alkyloxy; or R₁₀ is joined to Y so that Y-R₁₀ is N-B=N where B is N or CH; and
- R₁₁: is hydrogen, halo, C₁₋₆ alkoxy or C₁₋₆ alkyl; or
- R₁₀ and R₁₁: are joined to form -OCH(R₁₅R₁₆)-E- wherein E is (CH₂)ₙ or NR₁₇CO(CH₂)m wherein n is 1 or 2 and m is 0 or 1 and R₁₅, R₁₆ and R₁₇ are independently selected from hydrogen or C₁₋₆ alkyl;
- R₁₂: is hydrogen or C₁₋₆ alkoxy or; amino optionally substituted by a C₁₋₆ alkyl group; or R₁₂ is C₁₋₇ alkanoylamino; and
- R₁₃: is halo, C₁₋₆ alkyl, C₁₋₆ alkoxy or C₁₋₆ alkylthio;
- R₁₄: is hydrogen or C₁₋₆ alkyl; and
- L: is CH or N.

Z is often benzyl, n- or iso-butyl, n- or iso-propyl, ethyl or methyl, preferably isopropyl or ethyl.

Examples of moieties in alkyl or alkyl containing groups in Z or in R₁ to R₁₄ include methyl, ethyl, n- and iso-propyl, n-, iso-, sec- and tert-butyl, preferably methyl. Cycloalkyl moieties include C₃, C₄, C₅, C₆, C₇ and C₈ cycloalkyl. Halo moieties or "halo" include fluoro, chloro, bromo and iodo.

Suitable examples of R₂ and R₄ or R₈ and R₉ when joined include C₂, C₃, C₄, C₅ or C₆ polymethylene, preferably C₂, C₃, C₄ or C₅ polymethylene.

Rₐ to Rₑ and R_{g} to Rₕ are preferably selected from hydrogen, fluoro, chloro and hydroxy, most preferably hydrogen, R_{b} may be 5-, 6- or 7-chloro or fluoro.

When X₁ is of sub-formula (a), one of R₁ and R₃ is preferably hydrogen and one or both of R₂ and R₄ (most preferably both) are alkyl groups, such as methyl, or are joined to form C₂₋₇ polymethylene; or when one of R₂ and R₄ is hydrogen, the other is preferably ethyl or n- or iso- propyl.

When X₁ is of sub-formula (b), R₅ is preferably hydrogen or a methyl or ethyl group.

When X₁ is of sub-formula (c), one of CO-Y and R₆ is attached at the 1-position and the other is attached at the 3-position as depicted in sub-formula (c), and R₆ is preferably methyl or ethyl.

When X₁ is of sub-formula (d), R₇ is preferably methyl.

When X₁ is of sub-formula (e), R₈ and R₉ are preferably both methyl groups.

When X₁ is of sub-formula (f), and R₁₀ is C₁₋₆ alkoxy or is joined to Y, R₁₂ is preferably amino and R₁₃ is preferably chloro or bromo, most preferably chloro. R₁₀ is preferably methoxy when C₁₋₆ alkoxy.

When X₁ is of sub-formula (f), and R₁₀ is hydrogen, R₉ and R₁₁ are preferably chloro or methyl and R₁₀ is preferably hydrogen.

Other values of X₁ within sub-formula (f) of interest are those described in EP-A-307172 (Eli Lilly and Company) and EP-A-313393 (Yoshitomi Pharmaceutical Industries Limited).

When X₁ is of sub-formula (g), R₁₄ is preferably hydrogen or methyl.

When X₁ is fused, and -CO-Y- is attached to X₁ at an aromatic carbon atom, it is preferably attached at the aromatic carbon adjacent a 'fused' carbon atom, which is attached to the heteroatom of a heterocyclic ring in formula (IA).

Suitable examples of X₁ are as described in the aforementioned patent publications relating to 5-HT₃ receptor antagonists.

For the avoidance of doubt, the suitable X₁ values in formula (IA) which are described in the referenced patent publications, are that part of the structure remaining when the saturated azabicyclic moiety and -CO-Y- are disregarded.

Y is preferably NH.

The pharmaceutically acceptable salts of the compounds of the formula (IA) include acid addition salts with conventional acids such as hydrochloric, hydrobromic, boric, phosphoric, sulphuric acids and pharmaceutically acceptable organic acids such as acetic, tartaric, maleic, citric, succinic, benzoic, ascorbic, methanesulphonic, α-keto glutaric, α-glycerophosphoric, and glucose-1-phosphoric acids.

The pharmaceutically acceptable salts of the compounds of the formula (IA) are usually acid addition salts with acids such as hydrochloric, hydrobromic, phosphoric, sulphuric, citric, tartaric, lactic and acetic acid.

Preferably the acid addition salt is the hydrochloride salt.

Examples of pharmaceutically acceptable salts include quaternary derivatives of the compounds of formula (IA) such as the compounds quaternised by compounds Rₓ-T wherein Rₓ is C₁₋₆ alkyl, phenyl-C₁₋₆ alkyl or C₅₋₇ cycloalkyl, and T is a radical corresponding to an anion of an acid. Suitable examples of Rₓ include methyl, ethyl and n- and iso-propyl; and benzyl and phenethyl. Suitable examples of T include halide such as chloride, bromide and iodide.

Examples of pharmaceutically acceptable salts also include internal salts such as N-oxides.

It will be appreciated that mono- or di- salts may be formed owing to the presence of two salifiable nitrogens in the azagranatane side chain.

The compounds of the formula (IA), their pharmaceutically acceptable salts, (including quaternary derivatives and N-oxides) may also form pharmaceutically acceptable solvates, such as hydrates, which are included wherever a compound of formula (IA) or a salt thereof is herein referred to.

It will of course be realised that some of the compounds of the formula (IA) have chiral or prochiral centres and thus are capable of existing in a number of stereoisomeric forms including enantiomers. The invention extends to each of these stereoisomeric forms (including enantiomers), and to mixtures thereof (including racemates). The different stereoisomeric forms may be separated one from the other by the usual methods.

The invention also provides a process for the Preparation of a compound of formula (IA), or a pharmaceutically acceptable salt thereof, which process comprises reacting a compound of formula (IV):

X₁'-COQ₁ (IV)

with a compound of formula (V): 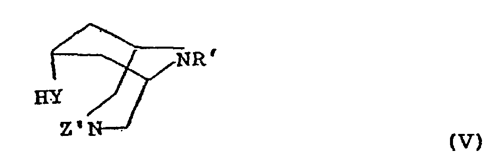 or a reactive derivative thereof, when Y is O;
wherein X₁' is X₁ or a group convertible thereto; Q₁ is a leaving group; R' and Z' are R and Z as defined or a hydrogenolysable protecting group; and the remaining variables are as hereinbefore defined; and thereafter optionally converting X₁' to X₁, including any Rₐ, R_{b}, R_{c}, R_{d}, Rₑ, R_{g}, Rₕ or R₁₀, R₁₁, R₁₂, R₁₃ or R₁₄ group to another such group, converting R'/Z', when other than R/Z, to R/Z; and
optionally forming a pharmaceutically acceptable salt of the resultant compound of formula (IA).

Intermediates of the formula (IV) (X₁'-COQ₁) are generally known from the aforementioned patent publications/references, or are prepared by analogous methods to those used for structurally related known compounds.

Intermediates of the formula (V) are generally prepared from the compound of formula (III): 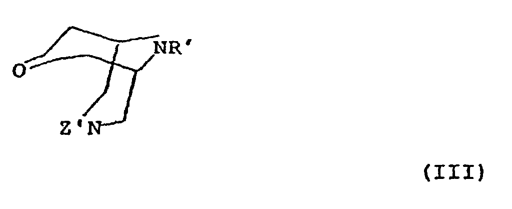 which is prepared by the condensation/cyclisation of as appropriate 2,6-disubstituted piperazine derivative, as described in the descriptions hereinafter.

Examples of leaving groups Q₁, displaceable by a nucleophile, include halogen such as chloro and bromo, C₁₋₄ alkoxy, such as CH₃O and C₂H₅O-, PhO-, or activated hydrocarbyloxy, such as Cl₅C₆O- or Cl₃CO-.

If a group Q₁ is a halide, then the reaction is preferably carried out at non-extreme temperatures in an inert non-hydroxylic solvent, such as benzene, dichloromethane, toluene, diethyl ether, tetrahydrofuran (THF) or dimethylformamide (DMF). It is also preferably carried out in the presence of an acid acceptor, such as an organic base, in particular a tertiary amine, such as triethylamine, trimethylamine, pyridine or picoline, some of which can also function as the solvent. Alternatively, the acid acceptor can be inorganic, such as calcium carbonate, sodium carbonate or potassium carbonate. Temperatures of 0°-100°C, in particular 10-80°C are suitable.

If a group Q₁ is C₁₋₄ alkoxy, phenoxy or activated hydrocarbyloxy then the reaction is preferably carried out in an inert polar solvent, such as toluene or dimethylformamide. It is also preferred that the group Q₁ is Cl₃CO- and that the reaction is carried out in toluene at reflux temperature.

When Y is O the compound of formula (V) may be in the form of a reactive derivative thereof, which is often a salt, such as the lithium, sodium or potassium salt.

Usually, X₁' will be X₁, but when R₁₀ is joined to Y, in formula (IA), X₁' is of sub-formula (f) wherein R₁₀ is nitro or amino, which may be subsequently be linked to Y as described in EP-A-315390.

It will be apparent that compounds of the formula (IA) containing an Rₐ to Rₑ, R_{g}, Rₕ or R₁₀ to R₁₄ group which is convertible to another such group are useful novel intermediates. i.e. a hydrogen substituent is convertible to a halogen substituent by halogenation using conventional halogenating agents; or a C₁₋₇ alkanoylamino substituent is convertible to amino by conventional hydrolysis.

R'/Z' when other than R/Z may be a hydrogenolysable protecting group which is benzyl optionally substituted by one or two groups selected from halo, C₁₋₄ alkoxy and C₁₋₄ alkyl. Such benzyl groups may, for example, be removed, when Rₐ to Rₑ, R_{g}, Rₕ, R₁₁ to R₁₄ is not halogen, by conventional transition metal catalysed hydrogenolysis to give the corresponding compound wherein R'/Z' is hydrogen.

A further process for the preparation of a compound of the formula (IA) or a pharmaceutically acceptable salt thereof, therefore comprises N-alkylating a compound of formula (IA), wherein R/Z is hydrogen and optionally forming a pharmaceutically acceptable salt of the resulting compound of the formula (IA). In this further process of the invention 'N-alkylation' comprises the substitution of the azabicyclic N-atoms by a group R/Z as hereinbefore defined. This may be achieved by reaction with a compound RQ₃ or ZQ₃ wherein R and Z are as hereinbefore defined and Q₃ is a leaving group. Suitable values for Q₃ include groups displaced by nucleophiles such as C1, Br, I, OSO₂CH₃ or OSO₂C₆H₄pCH₃. Favoured values for Q₃ include C1, Br and I. The reaction may be carried out under conventional alkylation conditions for example in an inert solvent such as dimethylformamide in the presence of an acid acceptor such as potassium carbonate. Generally the reaction is carried out at non-extreme temperature such as at ambient or slightly above. Alternatively, 'N-alkylation' may be effected under conventional reductive alkylation conditions.

Interconverting R or Z in the compound of the formula (V) before coupling with the compound of the formula (IV) is also possible. Such interconversions are effected conveniently under the above conditions. It is desirable to protect any amine function with a group readily removable by acidolysis such as a C₂₋₇ alkanoyl group, before R/Z interconversion.

It is often convenient in the preparation of such a compound of formula (V) to prepare the corresponding compound wherein the methylene group is replaced by -CO-, or for R or Z is methyl, where the methyl group is replaced by alkoxycarbonyl. Such compounds may then be reduced using a strong reductant such as lithium aluminium hydride to the corresponding compound of formula (IV).

The compounds of formula (IV) are known or are preparable analogously to, or routinely from, known compounds.

Compounds of the formula (V) wherein R' is R and Z' is Z as defined, are novel and form an aspect of the invention.

Compounds of the formula (IA) may also be prepared by the processes analogous to those described in the aforementioned European Patent Publications.

It will be realised that in the compound of the formula (IA) the -CO-Y- linkage has an endo orientation with respect to the ring of the bicyclic moiety to which it is attached. A mixture of endo and exo isomers of the compound of the formula (IA) may be synthesised non-stereospecifically and the desired isomer separated conventionally therefrom e.g. by chromatography; or alternatively the endo isomer-may if desired by synthesised from the corresponding endo form of the compound of the formula (II).

Pharmaceutically acceptable salts of the compounds of this invention may be formed conventionally.

The salts may be formed for example by reaction of the base compound of formula (IA) with a pharmaceutically acceptable organic or inorganic acid.

The compounds of the present invention are 5-HT₃ receptor antagonists and it is thus believed may generally be used in the treatment or prophylaxis of pain, emesis, CNS disorders and gastrointestinal disorders. Pain includes migraine, cluster headache, trigeminal neuralgia and visceral pain; emesis includes, in particular, that of preventing vomiting and nausea associated with cancer therapy, post-operative emesis, and nausea associated with migraine. Examples of such cancer therapy include that using cytotoxic agents, such as platinum complexes including cisplatin, and also doxorubicin and cyclophosphamide, particularly cisplatin; and also radiation treatment. CNS disorders include anxiety, psychosis, cognitive disorders such as senile dementia and age associated memory impairment (AAMI), and drug dependence. Gastrointestinal disorders include irritable bowel syndrome and diarrohea.

5-HT₃ receptor antagonists may also be of potential use in the treatment of obesity and/or arrhythmia.

Some of the compounds of the invention may also have gastric prokinetic activity, useful in the treatment of gastrointestinal disorders, for example when R₁₄ is C₁₋₆ alkyl.

The invention also provides a pharmaceutical composition comprising a compound of formula (IA), or a pharmaceutically acceptable salt thereof, and a pharmaceutically acceptable carrier.

Such compositions are prepared by admixture and are usually adapted for oral or parenteral administration, and as such may be in the form of tablets, capsules, oral liquid preparations, powders, granules, lozenges, reconstitutable powders, injectable and infusable solutions or suspensions or suppositories. Orally administrable compositions are preferred, since they are more convenient for general use.

Tablets and capsules for oral administration are usually presented in a unit dose, and contain conventional excipients such as binding agents, fillers, diluents, tabletting agents, lubricants, disintegrants, colourants, flavourings, and wetting agents. The tablets may be coated according to well known methods in the art, for example with an enteric coating.

Suitable fillers for use include cellulose, mannitol, lactose and other similar agents. Suitable disintegrants include starch, polyvinylpolypyrrolidone and starch derivatives such as sodium starch glycollate. Suitable lubricants include, for example, magnesium stearate.

Suitable pharmaceutically acceptable wetting agents include sodium lauryl sulphate. Oral liquid preparations may be in the form of, for example, aqueous or oily suspensions, solutions, emulsions, syrups, or elixirs, or may be presented as a dry product for reconstitution with water or other suitable vehicle before use. Such liquid preparations may contain conventional additives such as suspending agents, for example sorbitol, syrup, methyl cellulose, gelatin, hydroxyethylcellulose, carboxymethylcellulose, aluminium stearate gel or hydrogenated edible fats, emulsifying agents, for example lecithin, sorbitan monooleate, or acacia; non-aqueous vehicles (which may include edible oils), for example, almond oil, fractionated coconut oil, oily esters such as esters of glycerine, propylene glycol, or ethyl alcohol; preservatives, for example methyl or propyl p-hydroxybenzoate or sorbic acid, and if desired conventional flavouring or colouring agents.

Oral liquid preparations are usually in the form of aqueous or oily suspensions, solutions, emulsions, syrups, or elixirs or are presented as a dry product for reconstitution with water or other suitable vehicle before use. Such liquid preparations may contain conventional additives such as suspending agents, emulsifying agents, non-aqueous vehicles (which may include edible oils), preservatives, and flavouring or colouring agents.

The oral compositions may be prepared by conventional methods of blending, filling or tabletting. Repeated blending operations may be used to distribute the active agent throughout those compositions employing large quantities of fillers. Such operations are, of course, conventional in the art.

For parenteral administration, fluid unit dose forms are prepared containing a compound of the present invention and a sterile vehicle. The compound, depending on the vehicle and the concentration, can be either suspended or dissolved. Parenteral solutions are normally prepared by dissolving the compound in a vehicle and filter sterilising before filling into a suitable vial or ampoule and sealing.
Advantageously, adjuvants such as a local anaesthetic, preservatives and buffering agents are also dissolved in the vehicle. To enhance the stability, the composition can be frozen after filling into the vial and the water removed under vacuum.

Parenteral suspensions are prepared in substantially the same manner except that the compound is suspended in the vehicle instead of being dissolved and sterilised by exposure of ethylene oxide before suspending in the sterile vehicle. Advantageously, a surfactant or wetting agent is included in the composition to facilitate uniform distribution of the compound of the invention.

The invention further provides a method of treatment or prophylaxis of pain, emesis, CNS disorders and/or gastrointestinal disorders in mammals, such as humans, which comprises the administration of an effective amount of a compound of the formula (IA) or a pharmaceutically acceptable salt thereof.

An amount effective to treat the disorders hereinbefore described depends on the relative efficacies of the compounds of the invention, the nature and severity of the disorder being treated and the weight of the mammal. However, a unit dose for a 70kg adult will normally contain 0.05 to 1000mg for example 0.5 to 500mg, of the compound of the invention. Unit doses may be administered once or more than once a day, for example, 2, 3 or 4 times a day, more usually 1 to 3 times a day, that is in the range of approximately 0.0001 to 50mg/kg/day, more usually 0.0002 to 25 mg/kg/day.

No adverse toxicological effects are indicated at any of the aforementioned dosage ranges.

The invention also provides a pharmaceutical composition for use in the treatment and/or prophylaxis of pain, emesis, CNS disorders and/or gastrointestinal disorders which composition comprises an effect non-toxic amount of a compound of formula (IA) or a pharmaceutically acceptable salt thereof and pharmaceutically acceptable carrier.

The invention also provides a compound of formula (IA) or a pharmaceutically acceptable salt thereof for use as an active therapeutic substance, in particular for use in the treatment of pain, emesis, CNS disorders and/or gastrointestinal disorders.

The following Examples illustrate the preparation of compounds of formula (IA); the following descriptions illustrate the preparation of intermediates.

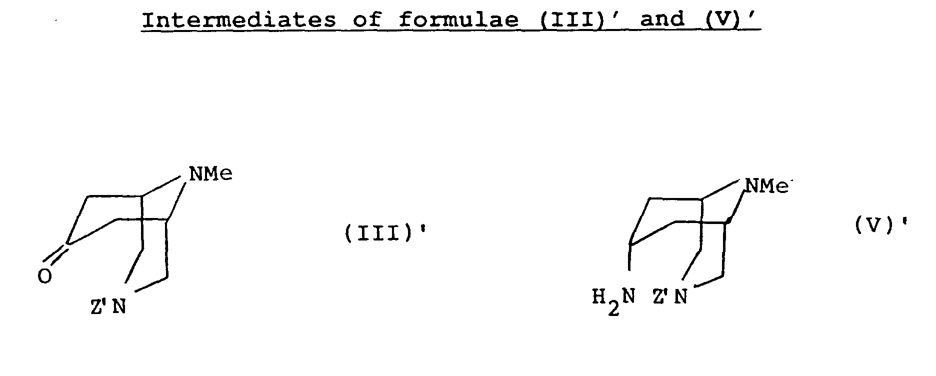

| | **Z'** |
|---|---|
| **D1c/d*** | CH₂Ph |
| **D2c/d** | CH₂Ph |
| **D3c/d** | ⁱPr |
| **D4c/d** | ⁿPr |
| **D5c/d** | ⁱBu |
| **D6c/d** | ⁿBu |
| **D7c/d** | Ph |
| **D8c/d** | Nm |
| **D9c/d** | Me |
| **D10c/d** | Et |
| **D11c/d** | Pe |
| **D12c/d** | Cm |
| | |
| Nm = 1-naphthylmethyl; | |
| Cm = cyclohexylmethyl; | |
| Pe = phenethyl | |

| | |
|---|---|
| * mixture of **endo** and **exo** isomers | |

### Description 1

### 3-Benzyl-9-methyl-3,9-diazabicyclo[3.3.1]nonan-7-amine

a) Ethyl 4-bromocrotonate (25g) in Et₂O (200 ml) was stirred, cooled to 0°C and benzylamine (12.6ml) in Et₂O (50ml) was added dropwise. The reaction was stirred at room temperature for 2 days, filtered and the filtrate washed with H₂O, dried (Na₂SO₄) and concentrated. Column chromatography of the residue on silica, eluting with 1:3 Et₂O:petrol gave diethyl-4,4¹-benzylimino di-trans-2-butenoate (15g).
b) The above diester (29g) dissolved in MeOH (300ml) at 0°C was treated with solution of 33% MeNH₂ in IMS (5.7ml). The reaction mixture was stirred at room temperature overnight, the solvent removed and the residue filtered through silica, eluting with 1:1 Et₂O:petrol to give dimethyl-4-benzyl-1-methyl-piperazinyl-2,6-diacetate (D1b) (13.9g).
c) The diester (13.9g) in toluene (150 ml) was added dropwise during 1h to a stirred suspension of Bu^{t}OK (12.9g) in toluene (600ml) being heated to reflux. After heating for a further 30 min., no starting material remained as determined by TLC. On cooling, the intermediate β-keto ester was extracted into 5N HCl (200ml) and the acidic extract heated to vigorous reflux for 5h. The reaction mixture was concentrated and the residue neutralised, then saturated with K₂CO₃ and the product extracted into CHCl₃. The concentrated organic extracts were purified by column chromatography on silica, eluting with 5% MeOH/CHCl₃ to give 3-benzyl-9-methyl-3,9-diazabicyclo[3.3.1]nonan-7-one (D1c) (3.7g)
d) The ketone (D1c) (2g) in EtOH (50ml) was heated to reflux with H₂NOH.HCl (0.65g) for 1h. The reaction mixture was cooled, concentrated to approx. 1/3rd volume, treated with a little ether and the hydrochloride salt of the oxime collected and dried (1.7g). The oxime hydrochloride (0.9g) was reduced with sodium (1.2g) in amyl alcohol (50ml) at reflux. After all the sodium had dissolved, the mixture was cooled to 90°C, water (4ml) was carefully added, then allowed to re-cool to room temperature. The aqueous layer was separated and the amyl alcohol extracted with an excess of 5N HCl. Concentration of the acidic extract, neutralisation then saturation with K₂CO₃ and extraction of the product into CHCl₃ gave, on concentration, the title compounds (D1d) (0.64g) as a crude mixture of **endo** and **exo** isomers.

### Description 2

### endo-3-Benzyl-9-methyl-3,9-diazabicyclo[3.3.1]nonan-7-amine

a) To methyl-4-bromocrotonate (50g) in diethyl ether (500ml) was added, dropwise benzylamine (22ml) in diethyl ether (20ml) at 0°C. The reaction mixture was stirred at room temperature for 72h. The precipitate was removed by filtration and the filtrate washed with water (75 ml). The organic phase was dried (MgSO₄), the solvent evaporated under reduced pressure and the residue purified using flash chromatography on silica eluting with light petrol and diethyl ether to afford dimethyl-4,4¹-benzyliminodi-**trans**-2-butenoate (17.1g).
b) To dimethyl-4,4¹-benzyliminodi**-trans**-2-butenoate (17.1g) in methanol (250 ml) was added dropwise methylamine (7.5 ml, 33% w/w in IMS) at 0°C. The reaction mixture was stirred overnight at room temperature. The solvent was evaporated under reduced pressure and the residue chromatographed on silica using light petrol and diethyl ether as the eluant to afford dimethyl-1-benzyl-4-methyl piperazinyl-3,5-diacetate as a mixture of **cis** and **trans** isomers (9.29g).
c) To potassium tert-butoxide (9.67g) in toluene (350 ml) was added dimethyl-4-benzyl-1-methyl piperazinyl-2,6-diacetate (9.26g) in toluene (150 ml) at room temperature under a nitrogen atmosphere. The reaction mixture was heated to reflux for 3h. The reaction mixture was cooled and washed with 5N HCl (4x75ml). The combined aqueous extracts were heated to reflux for 13h. The reaction mixture was cooled, the solvent concentrated under reduced pressure and the residue saturated with solid potassium carbonate. The product was extracted into chloroform (4x75ml), the organic phase dried (MgSO₄) and the solvent evaporated under reduced pressure. Flash chromatography on silica using chloroform with increasing volumes of ethanol (up to 10%) eluant gave 3-benzyl-9-methyl-3,9-diazabicyclo[3.3.1]nonan-7-one (D2c) (1.8g).
d) To a stirred solution of the above ketone (1.80g) in ethanol (50ml) was added hydroxylamine hydrochloride (0.54g). The reaction mixture was then heated to reflux for 2h. The reaction mixture was cooled and the solvent evaporated under reduced pressure. The residue was triturated with diethyl ether to give 3-benzyl-9-methyl-3,9-diazabicyclo[3.3.1]nonan-7-one oxime hydrochloride (1.87g).

To a stirred solution of alane [generated by the action of conc. H₂SO₄ (0.93ml) on lithium aluminium hydride (0.88g) in dry THF (30ml)] was added 3-benzyl-9-methyl-3,9-diazabicyclo[3.3.1]nonan-7-one oxime [generated by the treatment of 3-benzyl-9-methyl-3,9-diazabicyclo[3.3.1]nonan-7-one oxime hydrochloride with potassium carbonate). The reaction mixture was then heated to reflux overnight under a nitrogen atmosphere. The reaction mixture was cooled and 40% aqueous NaOH solution (2ml) and water (1ml) were added dropwise. Diethyl ether (5ml) was added and the mixture stirred for 1h. The resultant precipitate was removed by filtration through kieselguhr and the filtrate concentrated under reduced pressure to afford the crude title compound (D2d) (0.90g).

Following the procedures outlined in Descriptions 1 and 2, parts a) to c) the following intermediates were obtained:
3-isopropyl-9-methyl-3,9-diazabicyclo[3.3.1]nonan-7-one (D3c);
3-n-propyl-9-methyl-3,9-diazabicyclo[3.3.1]nonan-7-one (D4c);
3-isobutyl-9-methyl-3,9-diazabicyclo[3.3.1]nonan-7-one (D5c);
3-ⁿbutyl-9-methyl-3,9-diazabicyclo[3.3.1]nonan-7-one (D6c);
9-methyl-3-phenyl-3,9-diazabicyclo[3.3.1]nonan-7-one (D7c);
9-methyl-3-(1-naphthylmethyl)-3, 9-diazabicyclo[3.3.1]nonan-7-one (D8c) ;
3, 9-dimethyl-3,9-diazabicyclo[3.3.1]nonan-7-one (D9c);
3-ethyl-9-methyl-3,9-diazabicyclo[3.3.1]nonan-7-one (D10c);
9-methyl-3- (2-phenethyl)-3, 9-diazabicyclo[3.3.1]nonan-7-one (D11c) ;
3-cyclohexylmethyl-9-methyl-3,9-diazabicyclo[3.3.1]nonan-7-one (D12c).

Following the procedures outlined in descriptions 1d) and 2d) the following intermediates were obtained:
**endo**-3-isopropyl-9-methyl-3,9-diazabicyclo[3.3.1]nonan-7-amine (D3d);
**endo**-3-ⁿpropyl-9-methyl-3,9-diazabicyclo[3.3.1]nonan-7-amine (D4d);
**endo**-3-isobutyl-9-methyl-3,9-diazabicyclo[3.3.1]nonan-7-amine (D5d);
**endo**-3-ⁿbutyl-9-methyl-3,9-diazabicyclo[3.3.1]nonan-7-amine (D6d);
**endo**-9-methyl-3-phenyl-3,9-diazabicyclo[3.3.1]nonan-7-amine (D7d);
**endo**-9-methyl-3-(1-naphthylmethyl)-3,9-diazabicyclo[3.3.1]-nonan-7-amine (D8d);
**exo/endo**-3,9-dimethyl-3,9-diazabicyclo[3.3.1]nonan-7-amine (D9d) ;
**endo**-3-ethyl-9-methyl-3,9-diazabicyclo[3.3.1]nonan-7-amine (D10d);
**endo**-9-methyl-3-(2-phenethyl)-3,9-diazabicyclo[3.3.1]-nonan-7-amine (D11d);
**endo**-3-cyclohexylmethyl-9-methyl-3,9-diazabicyclo[3.3.1]-nonan-7-amine (D12d);

### Examples

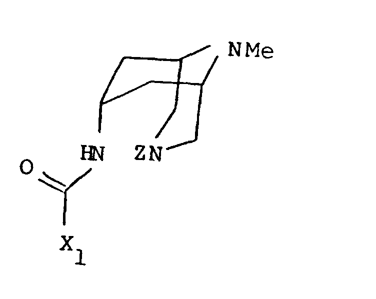

| | **X**_{**1**} | **Z** |
|---|---|---|
| **E1a/ E1b** | X₁ = (f) : R₁₀ = OCH₃, R₁₁ = H, R₁₂ = NHCOCH₃, NH₂, R₁₃ = Cl. | CH₂Ph |
| | | |
| **E2** | (as E1b) | Me |
| | | |
| **E3** | X₁ = (b): L = N, R₅ = CH₃, R_{b} = H. | CH₂Ph |
| | | |
| **E4** | (as E3) | i_{Pr} |
| | | |
| **E5** | (as E3) | n_{Pr} |
| | | |
| **E6** | (as E3) | i_{Bu} |
| | | |
| **E7** | (as E3) | n_{Bu} |
| | | |
| **E8** | (as E3) | Ph |
| **E9** | (as E3) | Nm |
| | | |
| **E10** | (as E3) | Et |
| | | |
| **E11** | (as E3) | Pe |
| | | |
| **E12** | (as E3) | Cm |
| | | |
| **E13** | X₁ = (a): R₁ = H, R₂ = Me, R₃ = H, R₄ = Me, Rₐ = H. | CH₂Ph |
| | | |
| **E14** | (as E13) | i_{Pr} |
| | | |
| **E15** | (as E13) | Me |
| | | |
| **E16** | (as E13) | Et |
| | | |
| **E17** | (as E13) | n_{Pr} |
| | | |
| **E18** | (as E13) | n_{Bu} |
| | | |
| **E19** | (as E13) | i_{Bu} |
| | | |
| **E20** | (as E13) | Cm |
| | | |
| **E21** | (as E13) | H |

### Example 1

### endo-4-Acetylamino-5-chloro-2-methoxy-N-(3-benzyl-9-methyl-3,9-diazabicyclo[3.3.1]nonan-7-yl)benzamide (E1a)

A solution of the crude amine (D1d) (0.64g) and Et₃N (0.4ml) in CH₂Cl₂ (20ml) was added to a stirred solution of 4-acetylamino-5-chloro-2-methoxybenzoyl chloride (0.75g) in CH₂Cl₂ (50ml) at 0°C. After stirring overnight the reaction mixture was washed with aqueous NaHCO₃, dried, filtered and concentrated. The residue was purified by column chromatography on alumina, eluting with 1:1 CHCl₃:petrol to give three fractions;
Fraction 1; 0.24g **exo** isomer
Fraction 2; 0.46g mixture of **endo/exo** isomers
Fraction 3; 0.23g **endo** isomer (mainly) compound El.

### endo-4-Amino-5-chloro-2-methoxy-N-(3-benzyl-9-methyl-3,9-diazabicyclo[3.3.1]nonan-7-yl)benzamide (E1b)

The mainly **endo** isomer (E1a), (fraction 3) (0.23g) was hydrolysed with 10% NaOH solution (1.0ml) in EtOH (20ml) and H₂O (5ml) heated for 2 hours. The solvent was removed and the residue extracted into CHCl₃. The organic extract was dried and concentrated. The residue was filtered through alumina, eluting with CHCl₃ to give the desired product E2 (0.12g) mp 145-162°C which contained ca. 20% of the **exo** isomer.
MS M⁺ 428, 430
¹H NMR (CDCl₃); δ: 9.8 (brd 1H), 8.15 (s, 0.2H), 7.7 (s, 0.8H), 7. 5-6.8 (m, 5H), 6.29 (s, 0.2H), 6.21 (s, 0.8H), 5.7-5.5 (m, 0.2H), 4.65-4.5 (m, 0.8H),4.35 (brs, 0.4H), 4.24 (brs, 1.6H), .86 (s, 0.6H), 3.78 (s 2.4H), 3.5 (s, 2H), 3.0-2.3 (m 9H including 2.5, s, 3H), 1.42 (3, 1.6H).

### Example 2

### endo-4-Amino-5-chloro-2-methoxy-N-(3,9-dimethyl-3,9-diazabicyclo-[3.3.1]nonan-7-yl)benzamide (E2)

The title compound was prepared in a similar manner to that described in Example 1, from 3,9-dimethyl-3,9-diazabicyclo[3.3.1]nonan-7-amine (D9d).

### Example 3

### endo-N-1-Methyl-3-indazolyl-(3-benzyl-9-methyl-3,9-diazabicyclo[3.3.1]nonan-7-yl)carboxamide (E3)

To a stirred suspension of 1-methyl-1H-indazole-3-carboxylic acid (0.50g) in CH₂Cl₂ (40ml) was added oxalyl chloride (0.25ml) and 3 drops of DMF. The reaction mixture was stirred at room temperature for 2h. The solvent was evaporated under reduced pressure to afford crude 1-methyl-1H-indazole-3-carbonyl chloride.

To a solution of 1-methyl-lH-indazole-3-carbonyl chloride (127 mg) in CH₂Cl₂ (20ml) was added a solution of **endo**-3-benzyl-9-methyl-3,9-diazabicyclo[3.3.1]nonan-7-amine (D2d) (160 mg) and triethylamine (90µl) in CH₂Cl₂ (5ml). The reaction mixture was stirred overnight at room temperature. The resulting solution was washed with saturated NaHCO₃ solution, dried (MgSO₄) and the solvent removed under reduced pressure to afford crude product. Flash chromatography on silica using chloroform and ethanol as the eluant gave the title compound (E3) (33mg) mp 173-176°.
¹H NMR (CD₃OD) 400 MHz; δ: 1.51 (d, 2H), 2.48 (s, 3H), 2.51-2.60 (m, 2H), 2.63 (dd, 2H), 2.80 (d, 2H), 2.87-2.92 (m, 2H), 3.92 (s, 5H), 4.50-4.59 (m, 1H), 7.09-7.18 (m, 3H), 7.29 (t, 1H), 7.35 (d, 2H), 7.47 (t, 1H), 7.57 (d, 2H), 8.22 (d, 1H).
M⁺ 403

### Examples 4-12

Following the general procedure outlined in Example 3, the following compounds were obtained:
**endo**-N-1-Methyl-3-indazolyl-(3-isopropyl-9-methyl-3,9-diazabicyclo[3.3.1]nonan-7-yl)carboxamide (E4)
   mp 180-184°C
   ¹H NMR (CDCl₃) 250 MHz; δ: 1.27 (d, 6H), 1.49 (d, 2H), 2.45-2.59 (m, 5H), 2.65-2.81 (m, 3H), 2.85 (d, 2H), 4.08 (s, 3H), 4.63-4.77 (m, 1H), 7.22-7.30 (m, 1H), 7.36-7.47 (m, 2H), 8.42 (d, 1H), 10.62 (d, 1H).
   M⁺ 355
**endo**-N-1-Methyl-3-indazolyl-(9-methyl-3-ⁿpropyl-3,9-diazabicyclo[3.3.1]nonan-7-yl)carboxamide (E5)
   mp 147-149°C
   ¹H NMR (CDCl₃) 250MHz; δ: 0.97 (t, 3H), 1.50 (d, 2H), 1.70-1.88 (m, 2H), 2.43-2.63 (m, 2H), 2.81-2.96 (m, 4H), 4.07 (s, 3H), 4.63-4.77 (m, 1H), 7.23-7.31 (m, 1H), 7.35-7.48 (m, 2H), 8.41 (d, 1H), 10.70 (d, 1H).
   M⁺ 355
**endo**-N-1-Methyl-3-indazolyl-(3-isobutyl-9-methyl-3,9-diazabicyclo[3.3.1]nonan-7-yl)carboxamide (E6)
   mp 107-109°C
   ¹H NMR (CDCl₃) 250 MHz; δ:0.89 (d, 6H), 1.58 (d, 2H), 1.95-2.10 (m, 1H), 2.40-2.74 (m, 9H), 2.87 (d, 2H), 2.92-3.05 (m, 2H), 4.09 (s, 3H), 4.68-4.81 (m, 1H), 7.23-7.31 (m, 1H), 7.38-7.48 (m, 2H), 8.34 (d, 1H), 10.25 (d, 1H).
   MH⁺ 370
**endo**-N-1-Methyl-3-indazolyl-(3-ⁿbutyl-9-methyl-3,9-diazabicyclo[3.3.1]nonan-7-yl)carboxamide (E7)
   mp 91-93°C
   ¹H NMR (CDCl₃) 250 MHz; δ: 0.93 (t, 3H), 1.30-1.44 (m, 2H), 1.52 (d, 2H), 1.62-1.78 (m, 2H), 2.47-2.68 (m, 9H), 2.88 (d, 2H), 2.91-2.99 (m, 2H), 4.06 (s, 3H), 4.65-4.78 (m, 1H), 7.22-7.30 (m, 1H), 35-7.47 (m, 2H), 8.41 (d, 1H), 10.67 (d, 1H).
   MH⁺ 370
**endo**-N-1-Methyl-3-indazolyl-(9-methyl-3-phenyl-3,9-diazabicyclo[3.3.1]nonan-7-yl)carboxamide (E8)
   mp 131-134°C
   ¹H NMR (CDCl₃) 250Hz; δ: 1.69 (d, 2H), 2.61 (s, 3H), 3.11-3.21 (m, 2H), 3.23-3.34 (m, 2H), 3.39 (s, 3H), 3.43-3.60 (m, 4H), 4.69-4.72 (m, 1H), 6.98 (t, 1H), 7.08 (d, 2H), 7.15-7.41 (m, 5H), 8.31 (d, 1H), 9.45 (d, 1H).
   M⁺ 389
**endo**-N-1-Methyl-3-indazolyl-(3-(1-naphthylmethyl)-9-methyl-3,9-diazabicyclo[3.3.1]nonan-7-yl)carboxamide (E9)
   MP 89-92°C
   ¹H NMR (CDCl₃) 250 MHz; δ: 1.55 (d, 2H), 2.44-2.61 (m, 5H), 2.71-2.82 (m, 2H), 2.83-3.00 (m, 4H), 3.68 (s, 3H), 4.45 (s, 2H), 4.68-4.79 (m, 1H), 7.17 (t, 1H), 7.24-7.53 (m, 5H), 7.64 (d, 1H), 7.71 (d, 1H), 7.82-7.91 (m, 1H), 8.20-8.28 (m, 1H), 8.43 (d, 1H), 10.87 (d, 1H).
   MH⁺ 454
**endo**-N-1-Methyl-3-indazolyl-(3-ethyl-9-methvl-3,9-diazabicyclo[3.3.1]nonan-7-yl)carboxamide (E10)
   mp 140-143°C
   ¹H NMR (CDCl₃) 250 MHz; δ: 1.30 (t, 3H), 1.52 (d, 2H), 2.45-2.70 (m, 9H), 2.85 (d, 2H), 2.95 (brs, 2H), 4.08 (s, 3H), 4.60-4.75 (m, H), 7.20-7.30 (m, H), 7.35-7.46 (m, 2H), 8.42 (d, H), 10.85 (d, H).
   MS M⁺ = 341
**endo**-N-1-Methyl-3-indazolyl-(9-methyl-3-phenethyl-3,9-diazabicyclo[3.3.1]nonan-7-yl)carboxamide (E11)
   mp 129-131°
   ¹H NMR (CDCl₃) 250 MHz; δ: 1.57 (d, 2H), 2.50-2.68 (m, 5H), 2.70-2.84 (m, 2H), 2.85-3.05 (m, 6H), 3.06-3.18 (m, 2H), 3.80 (s, 3H), 4.68-4.80 (m, H), 7.15-7.48 (m, 8H), 8.40 (d, H), 10.56 (d, H).
   MS M⁺ = 417
**endo**-N-1-Methyl-3-indazolyl-(3-cyclohexylmethyl-9-methyl-3,9-diazabicyclo[3.3.1]nonan-7-yl)carboxamide (E12)
   mp 82-85°C
   ¹H NMR (CDCl₃) 250 MHz; δ: 0.75-1.15 (m, 4H), 1.40-1.72 (m, 8H), 2.45 (d, 2H), 2.48-2.72 (m, 7H), 2.80 (d, 2H), 2.94 (brs, 2H), 4.10 (s, 3H), 4.62-4.79 (m, H), 7.20-7.32 (m, 2H) , 7.35-7.48 (m, 2H), 8.32 (d, H), 10.21 (d, H).
   MS (E1) M⁺ = 409

### Example 13

### endo-N-3,3-Dimethylindolin-1-yl(3-benzyl-9-methyl-3,9-diazabicyclo[3.3.1]nonan-7-yl)carboxamide (E13)

A solution of 3,3-dimethylindoline (1.5g) and triethylamine (1.42ml) in CH₂Cl₂ (15ml) was added dropwise to a cooled stirred solution of phosgene (9ml, 12.5% w/w in toluene) in CH₂Cl₂ (15ml). The reaction mixture was stirred for 1h at 0°C and then poured into pentane (100ml), washed with 5N sulphuric acid (5ml) and brine (5ml). The organic phase was dried (MgSO₄) and the solvent evaporated under reduced pressure to give crude 1-(2,3-dihydro-3,3-dimethyl)indolylcarbonyl chloride (1.7g).

To a stirred solution of 1-(2,3-dihydro-3,3-dimethyl)indolylcarbonyl chloride (771mg) in CH₂Cl₂ (15ml) at ambient temperature was added **endo**-3-benzyl-9-methyl-3,9-diazabicyclo[3.3.1]nonan-7-amine (D2d) (902mg) and triethylamine (512µl) in CH₂Cl₂ (15ml). The reaction mixture was stirred at room temperature overnight. The resulting solution was washed with aqueous NaHCO₃ solution, dried (MgSO₄) and the solvent removed by rotary evaporation.

Flash chromatography on silica using chloroform and ethanol as the eluant gave the title compound (E13) (360mg) mp 188-191°C.
¹H NMR (CDCl₃) 250MHz; δ: 1.29 (s, 6H), 1.48 (d, 2H), 2.40-2.57 (m, 5H), 2.62-2.78 (m, 4H), 2.83-2.90 (m, 2H), 3.53 (s, 2H), 3.70 (s, 2H), 4.39-4.42 (m, 1H), 6.90 (t, 1H), 7.05-7.46 (m, 8H), 7.81 (d, 1H), 8.78 (d, 1H).

### Examples 14-20

Following the general procedure outlined in Example 13, the following compounds were obtained; in the case of hydrochloride salts, by treatment of the free base with ethereal HCl.
**endo**-N-3,3-Dimethylindolin-1-yl(3-isopropyl-9-methyl-3,9-diazabicyclo[3.3.1]nonan-7-yl)carboxamide (E14)
   mp 109-111°C
   ¹H NMR (CDCl₃) 250MHz; δ: 1.05 (d, 6H), 1.33 (s, 6H), 1.47 (d, 2H), 2.41-2.75 (m, 10H), 2.88-2.96 (m, 2H), 3.65 (s, 2H), 4.24-4.38 (m, 1H), 6.90 (t, 1H), 7.06-7.19 (m, 2H), 7.80 (d, 1H), 8.58 (d, 1H).
   MH⁺ 371
**endo**-N-3,3-Dimethylindolin-1-yl(3,9-dimethyl-3,9-diaza-bicyclo[3.3.1]nonan-7-yl)carboxamide (E15)
   mp 173-175°
   ¹H NMR (CDCl₃) 400 MHz; δ: 1.34 (s, 6H), 1.45 (d, 2H), 2.31 (s, 3H), 2.40-2.50 (m, 2H), 2.52 (s, 3H), 2.55-2.65 (m, 2H), 2.69 (d, 2H), 2.88 (brs, 2H), 3.54 (s, 2H), 4.25-4.36 (m, H), 6.90 (t, H), 7.07 (d, H), 7.15 (t, H), 7.93 (d, H), 9.15 (d, H).
   MS M⁺=342
**endo**-N-3,3-Dimethylindolin-1-yl(3-ethyl-9-methyl-3,9-diazabicyclo[3.3.1]nonan-7-yl)carboxamide (E16)
   mp 150°
   ¹H NMR (CDCl₃) 250 MHz; δ: 1.05 (t, 3H), 1.35 (s, 6H), 1.46 (d, 2H), 2.40-2.60 (m, 9H), 2.75 (d, 2H), 2.90 (brs, 2H), 3.60 (s, 2H), 4.25-4.40 (m, H), 6.90 (t, H), 7.05-7.20 (m, 2H), 7.85 (d, H), 8.95 (d, H).
   MS MH⁺ = 357
**endo**-N-3,3-Dimethylindolin-1-yl(3-ⁿpropyl-9-methyl-3,9-diazabicyclo[3.3.1]nonan-7-yl)carboxamide hydrochloride (E17)
   mp 139-142°C
   ¹HNMR (CDCl₃) 250 MHz - Free base; δ: 0.80 (t, 3H), 1.32 (s, 6H), 1.45 (d, 2H), 1.62-1.79 (m, 2H), 2.26-2.46 (m, 2H), 2.47-2.57 (m, 7H), 2.74 (d, 2H), 2.82-2.89 (m, 2H), 3.58 (s, 2H), 4.21-4.33 (m, 1H), 6.89 (t, 1H), 7.01-7.19 (m, 2H), 7.89 (d, 1H), 9.02 (d, 1H).
   MH+ 371 (Free base)
**endo**-N-3,3-Dimethylindolin-1-yl(3-ⁿbutyl-9-methyl-3,9-diazabicyclo[3.3.1]nonan-7-yl)carboxamide hydrochloride (E18)
   mp 135-138°C
   ¹H NMR (CDCl₃) 250 MHz - Free base; δ: 0.85 (t, 3H), 1.15-1.52 (m, 12H), 2.30-2.56 (m, 9H), 2.76 (d, 2H), 2.83-2.92 (m, 2H), 3.59 (s, 2H), 4.23-4.37 (m, 1H), 6.60 (t, 1H), 7.06-7.20 (m, 2H), 7.80 (d, 1H), 9.00 (d, 1H).
   MH⁺ 385 (Free base)
**endo**-N-3,3-Dimethvlindolin-1-yl(3-isobutyl-9-methyl-3,9-diazabicyclo[3.3.1]nonan-7-yl)carboxamide hydrochloride (E19)
   mp 141-144°C
   ¹H NMR (CDCl₃) 250MHz (Free base); δ: 0.69 (d, 6H), 1.30 (s, 6H), 1.49 (d, 2H), 2.17 (d, 2H), 2.39-2.55 (m, 8H), 2.65-2.78 (m, 2H), 2.81-2.90 (m, 2H), 3.64 (s, 2H), 4.25-4.37 (m, 1H), 6.88 (t, 1H), 6.98-7.15 (d, 1H), 7.51 (d, 1H), 8.69 (d, 1H).
   MH⁺ 385 (Free base)
**endo**-N-3,3-Dimethylindolin-1-yl(3-cyclohexylmethyl-9-methyl-3,9-diazabicyclo[3.3.1]nonan-7-yl carboxamide hydrochloride (E20)
   mp 130°C
   ¹H NMR (CDCl₃) 250 MHz; δ: 0.55-0.80 (m, 2H), 0.85-1.38 (m, 8H, including 1.33 (s, 6H), 1.40-1.90 (m, 10H), 2.22 (d, 2H), 2.40-2.59 (m, 7H including 2.50 (s, 3H)), 2.74 (d, 2H), 2.85 (brs, 2H), 3.65 (s, 2H), 4.29-4.43 (m, H), 6.90 (t, H), 7.05-7.19 (m, 2H), 7.62 (d, H).
   MS M⁺ = 425

### Example 21

### endo-N-3,3-Dimethylindolin-1-yl-(9-methyl-3,9-diazabicyclo[3.3.1]nonan-7-yl)carboxamide (E21)

endo-N-3,3-Dimethylindolin-1-yl(3-benzyl-9-methyl-3,9-diazabicyclo[3.3.1]nonan-7-yl)carboxamide (E13) (350 mg) was hydrogenated at atmospheric pressure in methanol (50ml) over 5% Pd/C catalyst for 4h. The catalyst was removed by filtration and the filtrate evaporated to give the title compound (E21) (149mg).
mp 248-251°C
¹H NMR (CDCl₃) 250 MHz; δ: 1.35 (s, 6H), 1.68 (d, 2H), 2.50-2.67 (m, 3H), 2.75 (s, 3H), 2.99 (d, 2H), 3.17-3.26 (m, 2H), 3.58 (s, 2H), 3.61-3.71 (m, 2H), 4.37-4.49 (m, 1H), 6.89-6.97 (m, 1H), 7.06-7.20 (m, 2H), 7.88 (m, 1H).
M⁺ 329

### 5-HT₃ Receptor Antagonist Activity

Compounds are evaluated for antagonism of the von Bezold-Jarisch reflex evoked by 5-HT in the anaesthetised rat according to the following method:
Male rats 250-350g, are anaesthetised with urethane (1.25g/kg intraperitoneally) and blood pressure and heart rate are recorded as described by Fozard J.R. et al., J. Cardiovasc. Pharmacol. 2, 229-245 (1980). A submaximal dose of 5-HT (usually 6µg/kg) is given repeatedly by the intravenous route and changes in heart rate quantified. Compounds are given intravenously and the concentration required to reduce the 5-HT-evoked response to 50% of the control response (ED₅₀) is then determined.

## Claims (Claims for the following Contracting State(s): AT, BE, CH, LI, DE, DK, FR, GB, IT, LU, NL, SE)

1. A compound of formula (IA), or a pharmaceutically acceptable salt thereof: 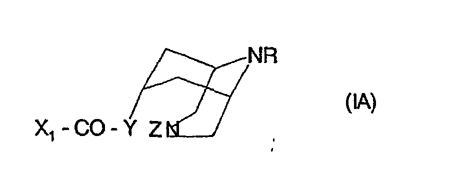 wherein
Z is C₁₋₆ alkyl, phenyl or phenyl C₁₋₄ alkyl wherein a phenyl moiety is optionally substituted by one or more of halo, C₁₋₆ alkoxy or C₁₋₆ alkyl;
R is methyl;
Y is NH or O (or is joined to R₁₀ as defined below); and
X₁ is a group of formula (a), (b), (c), (d), (e), (f) or (g): 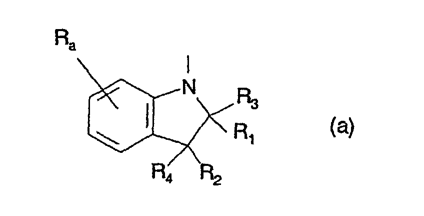 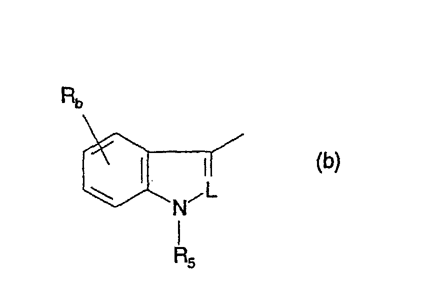 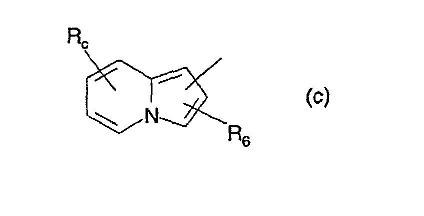 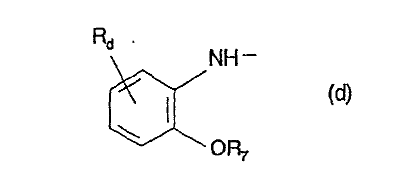 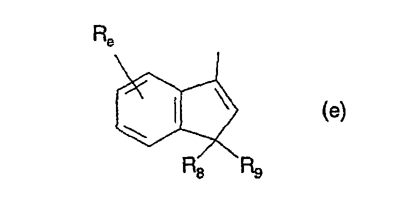 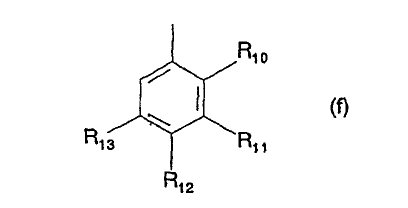 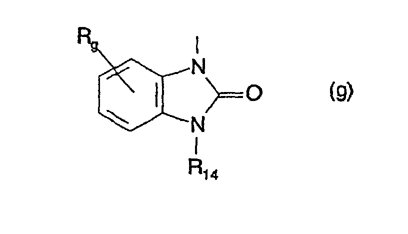
wherein
Rₐ to Rₑ and R_{g} are selected from hydrogen, halogen or hydroxy;
R₁ is hydrogen and R₂ is hydrogen or C₁₋₄ alkyl; or
R₁ and R₂ together are a bond;
R₃ to R₇ are independently hydrogen or C₁₋₆ alkyl; and
R₄ together with R₂ may be C₂₋₇ polymethylene or C₂₋₆ polymethylene interrupted by an -O- linkage when R₁ is hydrogen;
R₈ and R₉ are independently selected from hydrogen or C₁₋₆ alkyl or R₈ and R₉ together are C₂₋₆ polymethylene or C₂₋₅ polymethylene interrupted by an -O- linkage;
either R₁₀ is hydrogen, C₁₋₆ alkoxy, C₃₋₈ cycloalkyloxy or C₃₋₈ cycloalkyl C₁₋₄ alkyloxy; or R₁₀ is joined to Y so that Y-R₁₀ is N-B=N where B is N or CH; and
R₁₁ is hydrogen, halo, C₁₋₆ alkoxy or C₁₋₆ alkyl; or
R₁₀ and R₁₁ are joined to form -OCH(R₁₅R₁₆)-E- wherein E is (CH₂)ₙ or NR₁₇CO(CH₂)m wherein n is 1 or 2 and m is 0 or 1 and R₁₅, R₁₆ and R₁₇ are independently selected from hydrogen or C₁₋₆ alkyl;
R₁₂ is hydrogen or C₁₋₆ alkoxy or; amino optionally substituted by a C₁₋₆ alkyl group; or R₁₂ is C₁₋₇ alkanoylamino; and
R₁₃ is halo, C₁₋₆ alkyl, C₁₋₆ alkoxy or C₁₋₆ alkylthio;
R₁₄ is hydrogen or C₁₋₆ alkyl; and
L is CH or N.

2. A compound or salt according to claim 1 wherein X is of sub-formula (a), one of R₁ and R₃ is hydrogen, R₂ is C₁₋₄ alkyl and R₄ is C₁₋₆ alkyl or R₂ and R₄ are joined to form C₂₋₇ polymethylene.

3. A compound or salt according to claim 1 wherein X is of sub-formula (b), and R₅ is hydrogen or a methyl or ethyl group.

4. A compound or salt according to claim 1 wherein X is of sub-formula (d) and R₇ is methyl.

5. A compound or salt according to claim 1 wherein X is of sub-formula (f) wherein R₁₀ is methoxy, R₁₂ is amino and R₁₃ is chloro or bromo.

6. A compound or salt according to any one of claims 1 to 5, wherein Z is methyl.

7. A compound of formula (IB) or a pharmaceutically acceptable salt thereof: 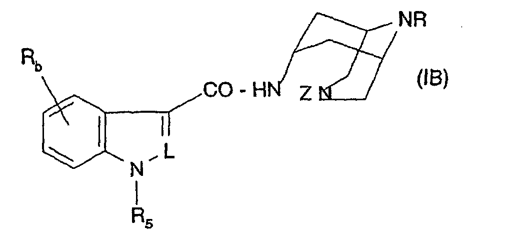 wherein
R is methyl;
R_{b} is H, OH or halogen;
R₅ is H, methyl or ethyl;
L is CH or N; and
Z is benzyl or methyl.

8. A compound or salt according to claim 7 wherein Z is methyl.

9. A compound according to claim 1 which is:
*endo*-4-amino-5-chloro-2-methoxy-N-(3-benzyl-9-methyl-3,9-diazabicyclo[3.3.1]nonan-7-yl)benzamide,
*endo*-4-amino-5-chloro-2-methoxy-N-(3,9-dimethyl-3,9-diazabicyclo-[3.3.1]nonan-7-yl)benzamide,
*endo*-N-1-methyl-3-indazolyl-(3-benzyl-9-methyl-3,9-diazabicyclo[3.3.1]nonan-7-yl)carboxamide,
*endo*-N-3,3-dimethylindolin-1-yl-(3-benzyl-9-methyl-3,9-diazabicyclo[3.3.1]nonan-7-yl)carboxamide,
or a pharmaceutically acceptable salt of any of the foregoing compounds.

10. A process for the preparation of a compound of formula (IA) according to any one of claims 1 to 8, or a pharmaceutically acceptable salt thereof, which process comprises:
(i) reacting a compound of formula (IV):
X₁'-COQ₁ (IV)
with a compound of formula (V): 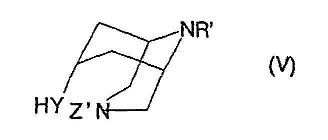 or a reactive derivative thereof, when Y is O;
wherein X₁' is X₁ or a group convertible thereto; Q₁ is a leaving group; R' and Z' are R and Z as defined in any one of claims I to 8 or a hydrogenolysable protecting group; and the remaining variables are as defined in any one of claims 1 to 8; and thereafter optionally converting X₁' to X₁, including any Rₐ, R_{b}, R_{c}, R_{d}, Rₑ, R_{g}, or R₁₀, R₁₁, R₁₂, R₁₃ or R₁₄ group to another such group; converting R'/Z', when other than R/Z, to R/Z; and optionally forming a pharmaceutically acceptable salt of the resultant compound of formula (IA);
or
(ii) N-alkylating a compound of formula (IA) except that R/Z is hydrogen, 'N-alkylation' comprising the substitution of the azabicyclic N-atoms by a group R/Z as defined in any one of claims 1 to 8; and optionally forming a pharmaceutically acceptable salt of the resulting compound of the formula (IA).

11. A compound of formula (V): 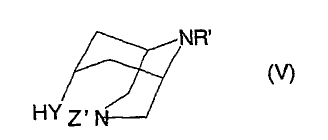 wherein R' is R and Z' is Z, as defined in any one of claims 1 to 8.

12. A compound according to claim 11 which is:
*endo-* and *exo-* 3-benzyl-9-methyl-3,9-diazabicyclo [3.3.1] nonan-7-amine or
*exo*/*endo*-3,9-dimethyl-3,9-diazabicyclo[3.3.1]nonan-7-amine.

13. A compound of formula (III) 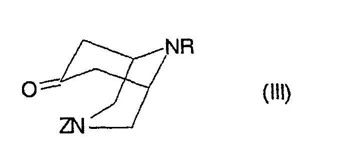 wherein Z and R are as defined in claim 1.

14. A compound according to claim 13 which is:
3-benzyl-9-methyl-3,9-diazabicyclo[3.3.1]nonan-7-one or
3,9-dimethyl-3,9-diazabicyclo[3.3.1]nonan-7-one.

15. A pharmaceutical composition comprising a compound or salt according to any one of claims 1 to 9, and a pharmaceutically acceptable carrier.

16. A compound or salt according to any one of claims 1 to 9, for use as an active therapeutic substance.

17. A compound or salt according to any one of claims 1 to 9, for use in the treatment of migraine, cluster headache, trigeminal neuralgia, visceral pain, emesis, CNS disorders or gastrointestinal disorders.

18. Use of a compound or salt according to any one of claims 1 to 9, in the manufacture of a medicament for the treatment of migraine, cluster headache, trigeminal neuralgia, visceral pain, emesis, CNS disorders or gastrointestinal disorders.

## Claims (Claims for the following Contracting State(s): ES, GR)

1. A process for the preparation of a compound of formula (IA), or a pharmaceutically acceptable salt thereof: 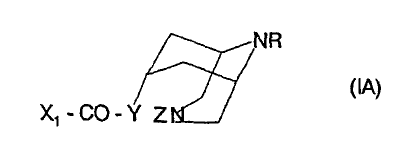 wherein
Z is C₁₋₆ alkyl, phenyl or phenyl C₁₋₄ alkyl wherein a phenyl moiety is optionally substituted by one or more of halo, C₁₋₆ alkoxy or C₁₋₆ alkyl;
R is methyl;
Y is NH or O (or is joined to R₁₀ as defined below); and
X₁ is a group of formula (a), (b), (c), (d), (e), (f) or (g): 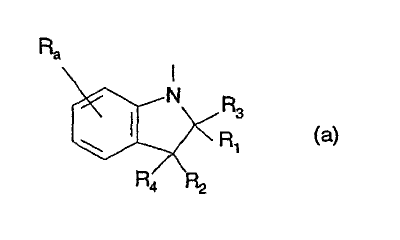 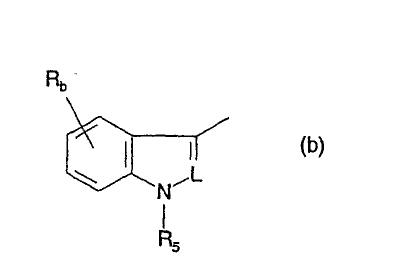 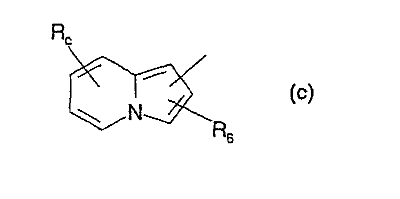 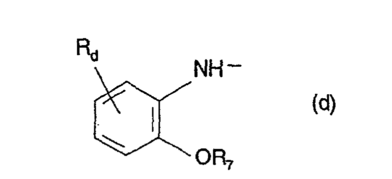 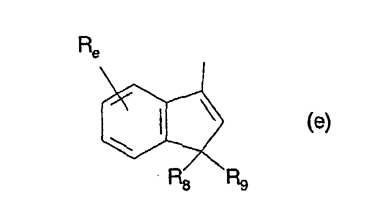 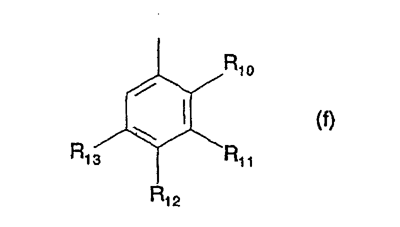 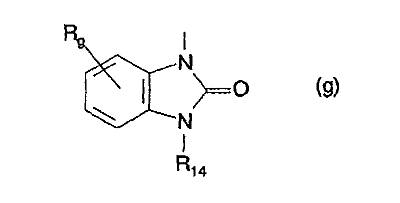
wherein
Rₐ to Rₑ and R_{g} are selected from hydrogen, halogen or hydroxy;
R₁ is hydrogen and R₂ is hydrogen or C₁₋₄ alkyl; or
R₁ and R₂ together are a bond;
R₃ to R₇ are independently hydrogen or C₁₋₆ alkyl; and
R₄ together with R₂ may be C₂₋₇ polymethylene or C₂₋₆ polymethylene interrupted by an -O- linkage when R₁ is hydrogen;
R₈ and R₉ are independently selected from hydrogen or C₁₋₆ alkyl or R₈ and R₉ together are C₂₋₆ polymethylene or C₂₋₅ polymethylene interrupted by an -O- linkage;
either R₁₀ is hydrogen, C₁₋₆ alkoxy, C₃₋₈ cycloalkyloxy or C₃₋₈ cycloalkyl C₁₋₄ alkyloxy; or R₁₀ is joined to Y so that Y-R₁₀ is N-B=N where B is N or CH; and
R₁₁ is hydrogen, halo, C₁₋₆ alkoxy or C₁₋₆ alkyl; or
R₁₀ and R₁₁ are joined to form -OCH(R₁₅R₁₆)-E- wherein E is (CH₂)ₙ or NR₁₇CO(CH₂)m wherein n is 1 or 2 and m is 0 or 1 and R₁₅, R₁₆ and R₁₇ are independently selected from hydrogen or C₁₋₆ alkyl;
R₁₂ is hydrogen or C₁₋₆ alkoxy or; amino optionally substituted by a C₁₋₆ alkyl group; or R₁₂ is C₁₋₇ alkanoylamino; and
R₁₃ is halo, C₁₋₆ alkyl, C₁₋₆ alkoxy or C₁₋₆ alkylthio;
R₁₄ is hydrogen or C₁₋₆ alkyl; and
L is CH or N;
which process comprises:
(i) reacting a compound of formula (IV):
X₁'-COQ₁ (IV)
with a compound of formula (V): 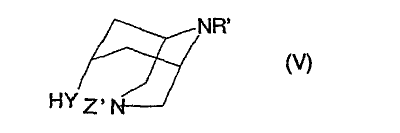 or a reactive derivative thereof, when Y is O;
wherein X₁' is X₁ or a group convertible thereto; Q₁ is a leaving group; R' and Z' are R and Z as defined or a hydrogenolysable protecting group; and the remaining variables are as hereinbefore defined; and thereafter optionally converting X₁' to X₁, including any Rₐ, R_{b}, R_{c}, R_{d}, Rₑ, R_{g}, or R₁₀, R₁₁, R₁₂, R₁₃ or R₁₄ group to another such group; converting R'/Z', when other than R/Z, to R/Z; and optionally forming a pharmaceutically acceptable salt of the resultant compound of formula (IA);
or
(ii) N-alkylating a compound of formula (IA) except that R/Z is hydrogen, 'N-alkylation' comprising the substitution of the azabicyclic N-atoms by a group R/Z as hereinbefore defined; and optionally forming a pharmaceutically acceptable salt of the resulting compound of the formula (IA).

2. A process according to claim 1 wherein X is of sub-formula (a), one of R₁ and R₃ is hydrogen, R₂ is C₁₋₄ alkyl and R₄ is C₁₋₆ alkyl or R₂ and R₄ are joined to form C₂₋₇ polymethylene.

3. A process according to claim 1 wherein X is of sub-formula (b), and R₅ is hydrogen or a methyl or ethyl group.

4. A process according to claim 1 wherein X is of sub-formula (d) and R₇ is methyl.

5. A process according to claim 1 wherein X is of sub-formula (f) wherein R₁₀ is methoxy, R₁₂ is amino and R₁₃ is chloro or bromo.

6. A process according to any one of claims 1 to 5, wherein Z is methyl.

7. A process as claimed in claim 1, wherein X₁ is a group of formula (b); Y is NH; R₅ is H, methyl or ethyl; and Z is benzyl or methyl.

8. A process according to claim 7, wherein Z is methyl.

9. A process according to claim 1 for the preparation of a compound of formula (I) which is:
*endo*-4-amino-5-chloro-2-methoxy-N-(3-benzyl-9-methyl-3,9-diazabicyclo[3.3.1]nonan-7-yl)benzamide,
*endo*-4-amino-5-chloro-2-methoxy-N-(3,9-dimethyl-3,9-diazabicyclo-[3.3.1]nonan-7-yl)benzamide,
*endo*-N-1-methyl-3-indazolyl-(3-benzyl-9-methyl-3,9-diazabicyclo[3.3.1]nonan-7-yl)carboxamide,
*endo*-N-3,3-dimethylindolin-1-yl-(3-benzyl-9-methyl-3,9-diazabicyclo[3.3.1]nonan-7-yl)carboxamide,
or a pharmaceutically acceptable salt of any of the foregoing compounds.

10. A process according to any one of claims 1 to 9 which is process (i) and wherein the Q₁ leaving group, displaceable by a nucleophile, is a halogen.

11. A process according to any one of claims 1 to 9 which is process (i) and wherein the Q₁ leaving group, displaceable by a nucleophile, is chloro or bromo.

12. A process according to claim 10 or 11 wherein the reaction is carried out at 0 to 100 °C in an inert non-hydroxylic solvent.

13. A process according to claim 12 wherein the reaction is carried out in the presence of an acid acceptor.

14. A process according to any one of claims 1 to 9 which is process (i) and wherein the Q₁ leaving group, displaceable by a nucleophile, is C₁₋₄ alkoxy, PhO-, Cl₅C₆O- or Cl₃CO-.

15. A process according to claim 14 wherein the reaction is carried out in an inert polar solvent.

16. A process according to claim 15 wherein the inert polar solvent is toluene or dimethylformamide.

17. A process according to any preceding claim which is process (i) and wherein R'/Z' are R/Z as defined in any of claims 1 to 8 or a hydrogenolysable protecting group which is benzyl optionally substituted by one or two groups selected from halo, C₁₋₄ alkoxy and C₁₋₄ alkyl.

18. A process according to any one of claims 1 to 9 which is process (ii) and wherein the N-alkylation is achieved by reaction with a compound RQ₃ or ZQ₃ wherein R and Z are as defined in any of claims 1 to 8 and Q₃ is a leaving group displaceable by nucleophiles.

19. A process according to claim 18 wherein the leaving group Q₃ is C1, Br, I, OSO₂CH₃ or OSO₂C₆H₄pCH₃.

## Patentansprüche (Patentansprüche für folgende(n) Vertragsstaat(en): AT, BE, CH, LI, DE, DK, FR, GB, IT, LU, NL, SE)

1. Verbindung der Formel (IA) oder ein pharmazeutisch annehmbares Salz davon: 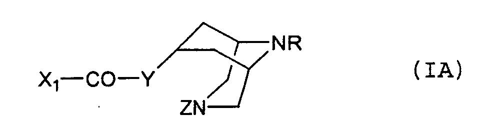 worin
Z ist C₁₋₆-Alkyl, Phenyl oder Phenyl-C₁₋₄-Alkyl, worin ein Phenylanteil gegebenenfalls durch ein oder mehrere Halogen, C₁₋₆-Alkoxy oder C₁₋₆-Alkyl substituiert ist; R ist Methyl;
Y ist NH oder O (oder ist verbunden mit R₁₀ wie nachstehend definiert); und
X₁ ist eine Gruppe der Formel (a), (b), (c), (d), (e), (f) oder (g): 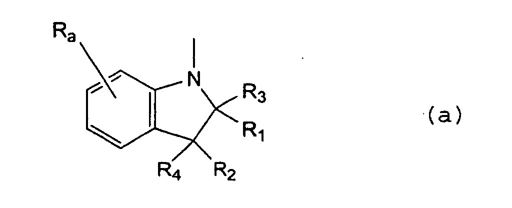 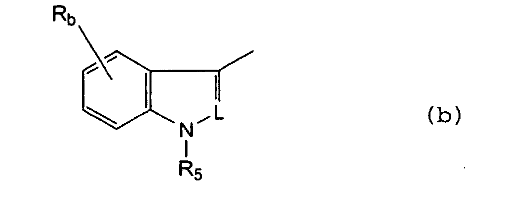 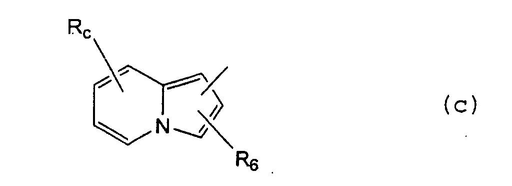 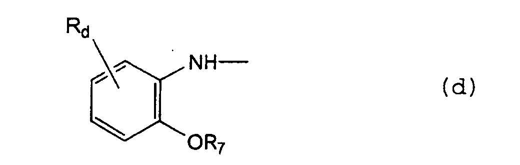 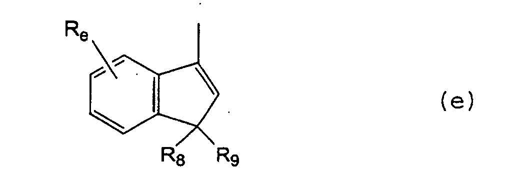 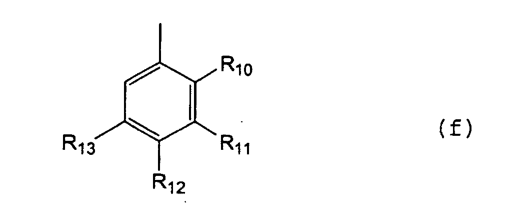 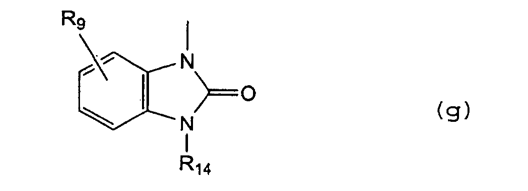 worin
Rₐ bis Rₑ und R_{g} ausgewählt sind aus Wasserstoff, Halogen oder Hydroxy;
R₁ ist Wasserstoff und R₂ ist Wasserstoff oder C₁₋₄-Alkyl; oder
R₁ und R₂ sind zusammen eine Bindung;
R₃ bis R₇ sind unabhängig Wasserstoff oder C₁₋₆-Alkyl; und
R₄ zusammen mit R₂ kann C₂₋₇-Polymethylen oder C₂₋₆-Polymethylen unterbrochen durch eine -O-Verknüpfung, wenn R₁ Wasserstoff ist, sein;
R₈ und R₉ sind unabhängig ausgewählt aus Wasserstoff oder C₁₋₆-Alkyl oder R₈ und R₉ sind zusammen C₂₋₆-Polymethylen oder C₂₋₅-Polymethylen unterbrochen durch eine -O-Verknüpfung;
entweder R₁₀ ist Wasserstoff, C₁₋₆-Alkoxy,
C₃₋₈-Cycloalkyloxy oder C₃₋₈-Cycloalkyl-C₁₋₄-Alkyloxy; oder R₁₀ ist mit Y verbunden, so daß Y-R₁₀ N-B=N ist, worin B N oder CH ist; und
R₁₁ ist Wasserstoff, Halogen, C₁₋₆-Alkoxy oder C₁₋₆-Alkyl; oder
R₁₀ und R₁₁ sind verbunden zur Bildung von -OCH(R₁₅R₁₆)-E-, worin E (CH₂)ₙ oder NR₁₇CO(CH₂)ₘ ist,
worin n 1 oder 2 und m 0 oder 1 ist, und R₁₅, R₁₆ und R₁₇ sind unabhängig ausgewählt aus Wasserstoff oder C₁₋₆-Alkyl;
R₁₂ ist Wasserstoff oder C₁₋₆-Alkoxy oder Amino gegebenenfalls substituiert durch eine C₁₋₆-Alkylgruppe; oder R₁₂ ist C₁₋₇-Alkanoylamino; und
R₁₃ ist Halogen, C₁₋₆-Alkyl, C₁₋₆-Alkoxy oder C₁₋₆-Alkylthio;
R₁₄ ist Wasserstoff oder C₁₋₆-Alkyl; und
L ist CH oder N.

2. Verbindung oder Salz gemäß Anspruch 1, worin X Unterformel (a) entspricht, eines aus R₁ und R₃ Wasserstoff ist, R₂ C₁₋₄-Alkyl ist und R₄ C₁₋₆-Alkyl ist, oder R₂ und R₄ sind verbunden zur Bildung von C₂₋₇-Polymethylen.

3. Verbindung oder Salz gemäß Anspruch 1, worin X Unterformel (b) entspricht und R₅ Wasserstoff oder eine Methyl- oder Ethylgruppe ist.

4. Verbindung oder Salz gemäß Anspruch 1, worin X Unterformel (d) entspricht und R₇ Methyl ist.

5. Verbindung oder Salz gemäß Anspruch 1, worin X Unterformel (f) entspricht, worin R₁₀ Methoxy ist, R₁₂ Amino ist und R₁₃ Chlor oder Brom ist.

6. Verbindung oder Salz gemäß einem der Ansprüche 1 bis 5, worin Z Methyl ist.

7. Verbindung der Formel (IB) oder ein pharmazeutisch annehmbares Salz davon: 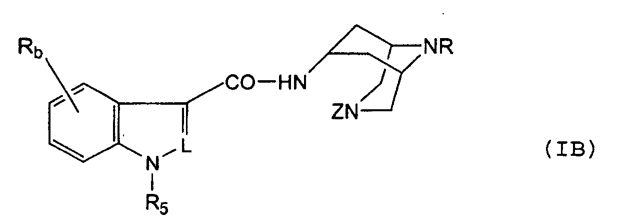 worin
R Methyl ist;
R_{b} H, OH oder Halogen ist;
R₅ H, Methyl oder Ethyl ist;
L CH oder N ist; und
Z Benzyl oder Methyl ist.

8. Verbindung oder Salz gemäß Anspruch 7, worin Z Methyl ist.

9. Verbindung gemäß Anspruch 1, bei der es sich handelt um:
endo-4-Amino-5-chlor-2-methoxy-N-(3-benzyl-9-methyl-3,9-diazabicyclo [3.3.1]nonan-7-yl)benzamid,
endo-4-Amino-5-chlor-2-methoxy-N-(3,9-dimethyl-3,9-diazabicyclo-[3.3.1]nonan-7-yl)benzamid,
endo-N-1-Methyl-3-indazolyl-(3-benzyl-9-methyl-3,9-diazabicyclo[3.3.1]nonan-7-yl)carboxamid,
endo-N-3,3-Dimethylindolin-1-yl-(3-benzyl-9-methyl-3,9-diazabicyclo [3.3.1]nonan-7-yl)carboxamid,
oder ein pharmazeutisch annehmbares Salz von einer der vorstehenden Verbindungen.

10. Verfahren zur Herstellung einer Verbindung der Formel (IA) gemäß einem der Ansprüche 1 bis 8 oder von einem pharmazeutisch annehmbaren Salz davon, worin das Verfahren umfaßt:
(i) Umsetzen einer Verbindung der Formel (IV) :
X₁'-COQ₁ (IV)
mit einer Verbindung der Formel (V): 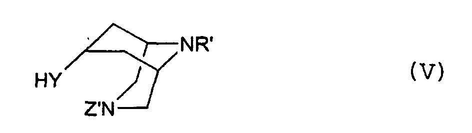 oder einem reaktiven Derivat davon, wenn Y O ist;
worin X₁' X₁ oder eine darin umwandelbare Gruppe ist; Q₁ eine Abgangsgruppe ist; R' und Z' R und Z wie in einem der Ansprüche 1 bis 8 definiert oder eine hydrierbare Schutzgruppe sind; und die verbleibenden Variablen wie in einem der Ansprüche 1 bis 8 definiert sind; und anschließend gegebenenfalls Umwandeln von X₁' in X₁, einschließlich einer der Rₐ, R_{b}, R_{c}, R_{d}, Rₑ, R_{g}, oder R₁₀, R₁₁, R₁₂, R₁₃ oder R₁₄ Gruppe in eine andere solche Gruppe; Umwandeln von R'/Z', wenn nicht R/Z, in R/Z; und gegebenenfalls Bilden eines pharmazeutisch annehmbaren Salzes der resultierenden Verbindung der Formel (IA) ;
oder
(ii) N-Alkylieren einer Verbindung der Formel (IA) außer daß R/Z Wasserstoff ist, worin "N-Alkylieren" die Substitution der azabicyclischen N-Atome durch eine Gruppe R/Z, wie in einem der Ansprüche 1 bis 8 definiert, umfaßt; und gegebenenfalls Bilden eines pharmazeutisch annehmbaren Salzes der resultierenden Verbindung der Formel (IA).

11. Verbindung der Formel (V): 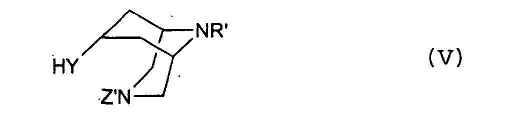 worin R' R ist und Z' Z ist, wie in einem der Ansprüche 1 bis 8 definiert.

12. Verbindung gemäß Anspruch 11, bei der es sich handelt um:
endo- und exo-3-Benzyl-9-methyl-3,9-diazabicyclo-[3.3.1]nonan-7-amin oder
exo/endo-3,9-Dimethyl-3,9-diazabicyclo-[3.3.1]nonan-7-amin.

13. Verbindung der Formel (III): 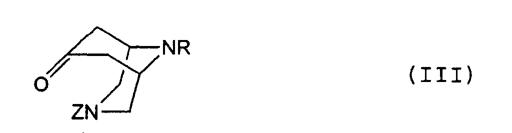 worin Z und R wie in Anspruch 1 definiert sind.

14. Verbindung gemäß Anspruch 13, bei der es sich handelt um:
3-Benzyl-9-methyl-3,9-diazabicyclo-[3.3.1]nonan-7-on oder
3,9-Dimethyl-3,9-diazabicyclo-[3.3.1]nonan-7-on.

15. Pharmazeutische Zusammensetzung umfassend eine Verbindung oder ein Salz gemäß den Ansprüchen 1 bis 9 und einen pharmazeutisch annehmbaren Träger.

16. Verbindung oder Salz gemäß einem der Ansprüche 1 bis 9 zur Verwendung als therapeutische Wirksubstanz.

17. Verbindung oder Salz gemäß einem der Ansprüche 1 bis 9 zur Verwendung in der Behandlung von Migräne, phasenhaft verlaufenden Kopfschmerzanfällen, Trigeminusneuralgie, viszeralem Schmerz, Erbrechen, ZNS-Störungen oder Störungen des Magendarmtrakts.

18. Verwendung einer Verbindung oder eines Salzes gemäß einem der Ansprüche 1 bis 9 für die Herstellung eines Arzneimittels für die Behandlung von Migräne, phasenhaft verlaufenden Kopfschmerzanfällen, Trigeminusneuralgie, viszeralem Schmerz, Erbrechen, ZNS-Störungen oder Störungen des Magendarmtrakts.

## Patentansprüche (Patentansprüche für folgende(n) Vertragsstaat(en): ES, GR)

1. Verfahren zur Herstellung einer Verbindung der Formel (IA) oder von einem pharmazeutisch annehmbares Salz davon: 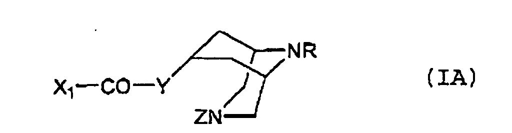 worin
Z ist C₁₋₆-Alkyl, Phenyl oder Phenyl-C₁₋₄-Alkyl, worin ein Phenylanteil gegebenenfalls durch ein oder mehrere Halogen, C₁₋₆-Alkoxy oder C₁₋₆-Alkyl substituiert ist; R ist Methyl;
Y ist NH oder O (oder ist verbunden mit R₁₀ wie nachstehend definiert); und
X₁ ist eine Gruppe der Formel (a), (b), (c), (d), (e), (f) oder (g): 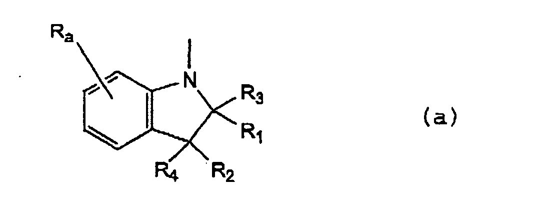 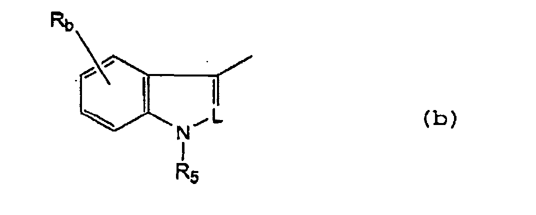 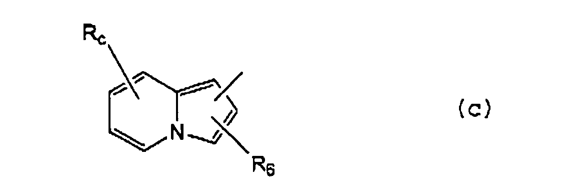 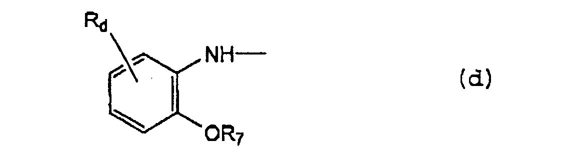 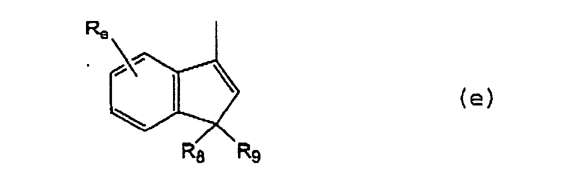 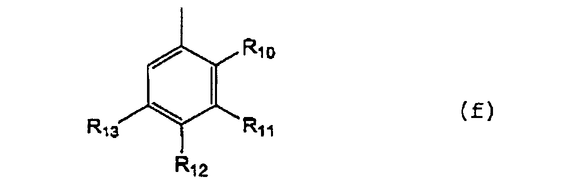 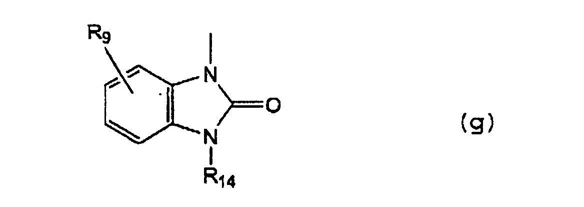 worin
Rₐ bis Rₑ und R_{g} ausgewählt sind aus Wasserstoff, Halogen oder Hydroxy;
R₁ ist Wasserstoff und R₂ ist Wasserstoff oder C₁₋₄-Alkyl; oder
R₁ und R₂ sind zusammen eine Bindung;
R₃ bis R₇ sind unabhängig Wasserstoff oder C₁₋₆-Alkyl; und
R₄ zusammen mit R₂ kann C₂₋₇-Polymethylen oder C₂₋₆-Polymethylen unterbrochen durch eine -O-Verknüpfung, wenn R₁ Wasserstoff ist, sein;
R₈ und R₉ sind unabhängig ausgewählt aus Wasserstoff oder C₁₋₆-Alkyl oder R₈ und R₉ sind zusammen C₂₋₆-Polymethylen oder C₂₋₅-Polymethylen unterbrochen durch eine -O-Verknüpfung;
entweder R₁₀ ist Wasserstoff, C₁₋₆-Alkoxy, C₃₋₈-Cycloalkyloxy oder C₃₋₈-Cycloalkyl-C₁₋₄-Alkyloxy; oder R₁₀ ist mit Y verbunden, so daß Y-R₁₀ N-B=N ist, worin B N oder CH ist; und
R₁₁ ist Wasserstoff, Halogen, C₁₋₆-Alkoxy oder C₁₋₆-Alkyl; oder
R₁₀ und R₁₁ sind verbunden zur Bildung von -OCH(R₁₅R₁₆)-E-, worin E (CH₂)n oder NR₁₇CO(CH₂)ₘ ist,
worin n 1 oder 2 und m 0 oder 1 ist, und R₁₅, R₁₆ und R₁₇ sind unabhängig ausgewählt aus Wasserstoff oder C₁₋₆-Alkyl;
R₁₂ ist Wasserstoff oder C₁₋₆-Alkoxy oder Amino gegebenenfalls substituiert durch eine C₁₋₆-Alkylgruppe; oder R₁₂ ist C₁₋₇-Alkanoylamino; und
R₁₃ ist Halogen, C₁₋₆-Alkyl, C₁₋₆-Alkoxy oder C₁₋₆-Alkylthio;
R₁₄ ist Wasserstoff oder C₁₋₆-Alkyl; und
L ist CH oder N;
worin das Verfahren umfaßt:
(i) Umsetzen einer Verbindung der Formel (IV):
X₁'-COQ₁ (IV)
mit einer Verbindung der Formel (V) : 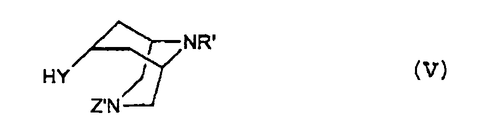 oder einem reaktiven Derivat davon, wenn Y O ist;
worin X₁' X₁ oder eine darin umwandelbare Gruppe ist; Q₁ eine Abgangsgruppe ist; R' und Z' sind R und Z wie definiert oder eine hydrierbare Schutzgruppe; und die verbleibenden Variablen sind wie vorstehend definiert; und anschließend gegebenenfalls Umwandeln von x₁' in X₁, einschließlich einer der Rₐ, R_{b}, R_{c}, R_{d}, Rₑ, R_{g}, oder R₁₀, R₁₁, R₁₂, R₁₃ oder R₁₄ Gruppe in eine andere solche Gruppe; Umwandeln von R'/Z', wenn nicht R/Z, in R/Z; und gegebenenfalls Bilden eines pharmazeutisch annehmbaren Salzes der resultierenden Verbindung der Formel (IA);
oder
(ii) N-Alkylieren einer Verbindung der Formel (IA) außer daß R/Z Wasserstoff ist, worin "N-Alkylieren" die Substitution der azabicyclischen N-Atome durch eine Gruppe R/Z, wie vorstehend definiert, umfaßt; und gegebenenfalls Bilden eines pharmazeutisch annehmbaren Salzes der resultierenden Verbindung der Formel (IA).

2. Verfahren gemäß Anspruch 1, worin X Unterformel (a) entspricht, eines aus R₁ und R₃ Wasserstoff ist, R₂ C₁₋₄-Alkyl ist und R4 C₁₋₆-Alkyl ist, oder R₂ und R₄ sind verbunden zur Bildung von C₂₋₇-Polymethylen.

3. Verfahren gemäß Anspruch 1, worin x Unterformel (b) entspricht und R₅ Wasserstoff oder eine Methyl- oder Ethylgruppe ist.

4. Verfahren gemäß Anspruch 1, worin X Unterformel (d) entspricht und R₇ Methyl ist.

5. Verfahren gemäß Anspruch 1, worin X Unterformel (f) entspricht, worin R₁₀ Methoxy ist, R₁₂ Amino ist und R₁₃ Chlor oder Brom ist.

6. Verfahren gemäß einem der Ansprüche 1 bis 5, worin Z Methyl ist.

7. Verfahren wie in Anspruch 1 beansprucht, worin X₁ eine Gruppe der Formel (b) ist; Y NH ist; R₅ H, Methyl oder Ethyl ist; und Z Benzyl oder Methyl ist.

8. Verfahren gemäß Anspruch 7, worin Z Methyl ist.

9. Verfahren gemäß Anspruch 1 zur Herstellung einer Verbindung der Formel (I), bei der es sich handelt um:
endo-4-Amino-5-chlor-2-methoxy-N- (3-benzyl-9-methyl-3,9-diazabicyclo [3.3.1]nonan-7-yl)benzamid,
endo-4-Amino-5-chlor-2-methoxy-N-(3,9-dimethyl-3,9-diazabicyclo-[3.3.1]nonan-7-yl)benzamid,
endo-N-1-Methyl-3-indazolyl-(3-benzyl-9-methyl-3,9-diazabicyclo-[3.3.1]nonan-7-yl)carboxamid,
endo-N-3,3-Dimethylindolin-1-yl-(3-benzyl-9-methyl-3,9-diazabicyclo [3.3.1]nonan-7-yl)carboxamid,
oder eines pharmazeutisch annehmbaren Salzes einer der vorstehenden Verbindungen.

10. Verfahren gemäß einem der Ansprüche 1 bis 9, das ein Verfahren (i) ist und worin die durch ein Nukleophil ersetzbare Q₁ Abgangsgruppe ein Halogen ist.

11. Verfahren gemäß einem der Ansprüche 1 bis 9, das ein Verfahren (i) ist und worin die durch ein Nukleophil ersetzbare Q₁ Abgangsgruppe Chlor oder Brom ist.

12. Verfahren gemäß Anspruch 10 oder 11, worin die Reaktionbei 0 bis 100°C in einem inerten nicht-hydroxyhaltigen Lösungsmittel durchgeführt wird.

13. Verfahren gemäß Anspruch 12, worin die Reaktion in Gegenwart eines Säureakzeptors durchgeführt wird.

14. Verfahren gemäß einem der Ansprüche 1 bis 9, das ein Verfahren (i) ist und worin die durch ein Nukleophil ersetzbare Q₁ Abgangsgruppe C₁₋₄-Alkoxy, PhO-, Cl₅C₆Ooder Cl₃CO- ist.

15. Verfahren gemäß Anspruch 14, worin die Reaktion in einem inerten polaren Lösungsmittel durchgeführt wird.

16. Verfahren gemäß Anspruch 15, worin das inerte polare Lösungsmittel Toluol oder Dimethylformamid ist.

17. Verfahren gemäß einem der vorstehenden Ansprüche, das ein Verfahren (i) ist und worin R'/Z' R/Z wie in einem der Ansprüche 1 bis 8 definiert oder eine hydrierbare Schutzgruppe sind, die Benzyl gegebenenfalls substituiert durch ein oder zwei Gruppen ausgewählt aus Halogen, C₁₋₄-Alkoxy und C₁₋₄-Alkyl ist.

18. Verfahren gemäß einem.der Ansprüche 1 bis 9, das ein Verfahren (ii) ist und worin die N-Alkylierung durch Reaktion mit einer Verbindung RQ₃ oder ZQ₃ erreicht wird, worin R und Z wie in einem der Ansprüche 1 bis 8 definiert sind und Q₃ eine durch Nukleophile ersetzbare Abgangsgruppe ist.

19. Verfahren gemäß Anspruch 18, worin die Abgangsgruppe Q₃ Cl, Br, I, OSO₂CH₃ oder OSO₂C₆H₄pCH₃ ist.

## Revendications (Revendications pour l'(les) Etat(s) contractant(s) suivant(s): AT, BE, CH, LI, DE, DK, FR, GB, IT, LU, NL, SE)

1. Composé de formule (IA) ou un sel de celui-ci acceptable d'un point de vue pharmaceutique : 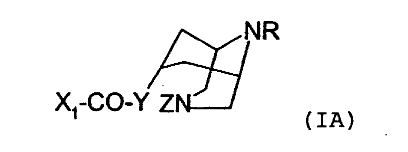 dans laquelle
Z est un groupe alkyle en C₁-C₆, un groupe phényle ou un groupe alkyle en C₁-C₄ substitué par un phényle dans lequel une chaîne phényle est facultativement substituée par un ou plusieurs groupes halo, alkoxy en C₁-C₆ ou alkyles en C₁-C₆ ;
R est un groupe méthyle ;
Y est NH ou O (ou est lié à R₁₀ tel que défini ci-dessous) ; et
X₁ est un groupe de formule (a), (b), (c), (d), (e), (f) ou (g) ; 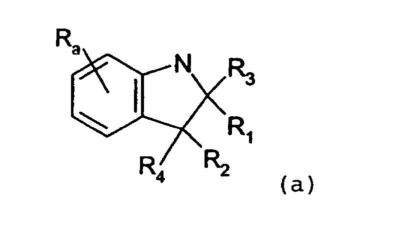 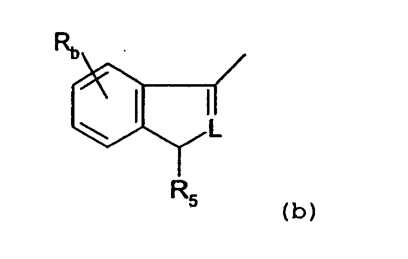 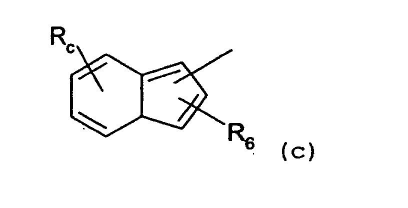 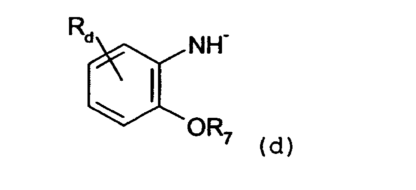 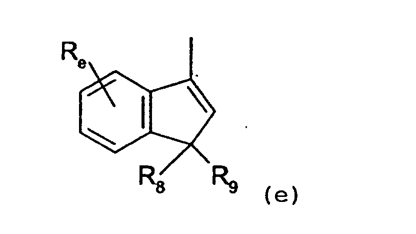 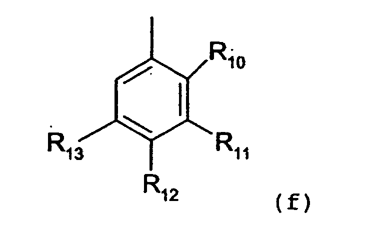 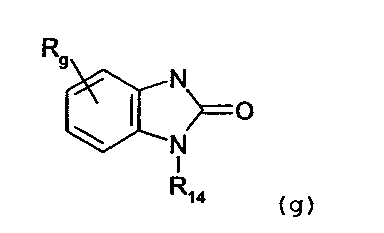 dans lesquelles
Rₐ à Rₑ et R_{g} sont choisis parmi l'hydrogène, les halogènes ou les groupes hydroxy ;
R₁ est un atome d'hydrogène et R₂ est un atome d'hydrogène ou un groupe alkyle en C₁-C₄ ; ou
R₁ et R₂ ensemble forment une liaison ;
R₃ à R₇ sont indépendamment un atome d'hydrogène ou un groupe alkyle en C₁-C₆ ; et
R₄ ensemble avec R₂ peut être un polyméthylène en C₂-C₇ ou un polyméthylène en C₂-C₆ interrompu par une liaison -O- lorsque R₁ est un atome d'hydrogène ;
R₈ et R₉ sont indépendamment choisis parmi l'hydrogène ou un groupe alkyle en C₁-C₆ ou R₈ et R₉ sont ensemble un polyméthylène en C₂-C₆ ou un polyméthylène en C₂-C₅ interrompu par une liaison -O- ;
soit R₁₀ est un atome d'hydrogène, un groupe alkoxy en C₁-C₆, un groupe cycloalkyloxy en C₃-C₈ ou un groupe alkyloxy en C₁-C₄ substitué par un groupe cycloalkyle en C₃-C₈ ; soit R₁₀ est lié à Y de sorte que Y-R₁₀ est N-B=N où B est N ou CH ; et
R₁₁ est un atome d'hydrogène, un groupe halo, un groupe alkoxy en C₁-C₆ ou un groupe alkyle en C₁-C₆ ; ou
R₁₀ et R₁₁ sont liés pour former -OCH(R₁₅R₁₆)-E- où E est (CH₂)ₙ ou NR₁₇CO(CH₂)ₘ où n est 1 ou 2 et m est 0 ou 1 et R₁₅, R₁₆ et R₁₇ sont indépendamment choisis parmi l'hydrogène ou un groupe alkyle en C₁-C₆ ;
R₁₂ est un atome d'hydrogène ou un groupe alkoxy en C₁-C₆ ou un groupe amino facultativement substitué par un groupe alkyle en C₁-C₆ ou R₁₂ est un groupe alkamoylamino ; et
R₁₃ est un groupe halo, un groupe alkyle en C₁-C₆, un groupe alkoxy en C₁-C₆ ou un groupe alkylthio en C₁-C₆ ;
R₁₄ est un atome d'hydrogène ou un groupe alkyle en C₁-C₆ ; et
L est CH ou N.

2. Composé ou sel selon la revendication 1 dans lequel X présente la sous-formule (a), un de R₁ et R₃ est un atome d'hydrogène, R₂ est un groupe alkyle en C₁-C₄ et R₄ est un groupe alkyle en C₁-C₆ ou R₂ et R₄ sont liés pour former un polyméthylène en C₂-C₇.

3. Composé ou sel selon la revendication 1 dans lequel X présente la sous-formule (b), et R₅ est un atome d'hydrogène ou un groupe méthyle ou éthyle.

4. Composé ou sel selon la revendication 1 dans lequel X présente la sous-formule (d) et R₇ est un groupe méthyle.

5. Composé ou sel selon la revendication 1 dans lequel X présente la sous-formule (f) dans laquelle R₁₀ est un groupe méthoxy, R₁₂ est un groupe amino et R₁₃ est un groupe chloro ou bromo.

6. Composé ou sel selon l'une quelconque des revendications 1 à 5 dans lequel Z est un groupe méthyle.

7. Composé de formule (IB) ou un sel de celui-ci acceptable d'un point de pharmaceutique : 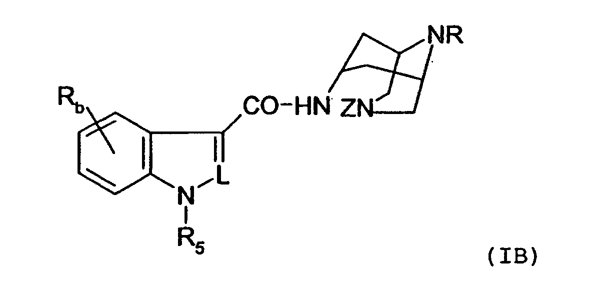 dans laquelle
R est un groupe méthyle ;
R_{b} est H, OH ou un halogène ;
R₅ est H, un groupe méthyle ou éthyle ;
L est CH ou N ; et
Z est un groupe benzyle ou méthyle.

8. Composé ou sel selon la revendication 7 dans lequel Z est un groupe méthyle.

9. Composé selon la revendication 1 qui est :
endo-4-amino-5-chloro-2-méthoxy-N-(3-benzyl-9-méthyl-3,9-diazabicyclo[3,3,1]nonan-7-yl)benzamide,
endo-4-amino-5-chloro-2-méthoxy-N-(3,9-diméthyl-3,9-diazabicyclo-[3,3,1]nonan-7-yl)benzamide,
endo-N-1-méthyl-3-indazolyl-(3-benzyl-9-méthyl-3,9-diazabicyclo[3,3,1]nonan-7-yl)carboxamide,
*endo*-N-3, 3-diméthylindolin-1-yl-(3-benzyl-9-méthyl-3,9-diazabicyclo[3,3,1]nonan-7-yl)carboxamide,
ou un sel acceptable d'un point de vue pharmaceutique d'un quelconque des composés mentionnés ci-dessus.

10. Procédé de préparation d'un composé de formule (IA) selon l'une quelconque des revendications 1 à 8, ou d'un sel de celui-ci acceptable d'un point de vue pharmaceutique, ledit procédé comprend :
(i) la réaction d'un composé de formule (IV) :
Z₁-COQ₁ (IV)
avec un composé de formule (V) : 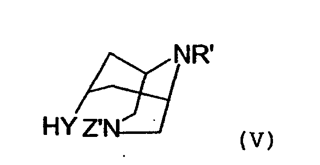 ou un dérivé réactif de celui-ci, lorsque Y est O ;
dans lesquelles X₁' est X₁ ou un groupe convertible en celui-ci ; Q₁ est un groupe partant ; R' et Z' sont R et Z tels que définis dans l'une quelconque des revendications 1 à 8 ou un groupe de protection hydrogénolysable et les variables restantes sont telles que définies dans l'une quelconque des revendications 1 à 8 ; et ensuite facultativement la conversion de X₁' en X₁, y compris n'importe quel groupe Rₐ, R_{b}, R_{c}, R_{d}, Rₑ, R_{g} ou R₁₀, R₁₁, R₁₂, R₁₃ ou R₁₄ en un autre tel groupe ; la conversion de R'/Z', lorsque différent de R/Z, en R/Z ; et facultativement la formation d'un sel acceptable d'un point de vue pharmaceutique du composé résultant de formule (IA) ;
ou
(ii) l'N-alkylation d'un composé de formule (IA) sauf que R/Z est un atome d'hydrogène, l'N-alkylation comprenant la substitution des N-atomes azabicycliques par un groupe R/Z tel que défini dans l'une quelconque des revendications 1 à 8 et facultativement la formation d'un sel acceptable d'un point de vue pharmaceutique du composé résultant de formule (IA).

11. Composé de formule (V) : 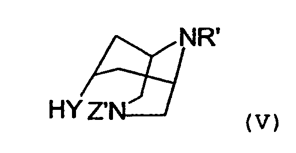 dans laquelle R' est R et Z' est Z, tels que définis dans l'une quelconque des revendications 1 à 8.

12. Composé selon la revendication 11 qui est :
*endo-* et *exo*-3-benzyl-9-méthyl-3,9-diazabicyclo-[3,3,1]nonan-7-amine ou
*exo*/*endo*-3,9-diméthyl-3,9-diazabicyclo[3,3,1]-nonan-7-amine.

13. Composé de formule (III) 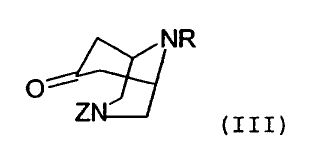 dans laquelle Z et R sont définis dans la revendication 1.

14. Composé selon la revendication 13 qui est :
3-benzyl-9-méthyl-3,9-diazabicyclo[3,3,1]nonan-7-one ou
3,9-diméthyl-3,9-diazabicyclo[3,3,1]nonan-7-one.

15. Composition pharmaceutique comprenant un composé ou un sel selon l'une quelconque des revendications 1 à 9 et un porteur acceptable d'un point de vue pharmaceutique.

16. Composé ou sel selon l'une quelconque des revendications 1 à 9 destiné à être utilisé comme substance thérapeutique active.

17. Composé ou sel selon l'une quelconque des revendications 1 à 9 destiné à être utilisé dans le traitement de la migraine, de la céphalée histaminique, la névralgie du trijumeau, la douleur viscérale, l'émésis, des troubles SNC ou des troubles gastro-intestinaux.

18. Utilisation d'un composé ou sel selon l'une quelconque des revendications 1 à 9 dans la production d'un médicament pour le traitement de la migraine, de la céphalée histaminique, la névralgie du trijumeau, la douleur viscérale, l'émésis, des troubles SNC ou des troubles gastro-intestinaux.

## Revendications (Revendications pour l'(les) Etat(s) contractant(s) suivant(s): ES, GR)

1. Procédé de préparation d'un composé de formule (IA) ou un sel de celui-ci acceptable d'un point de vue pharmaceutique : 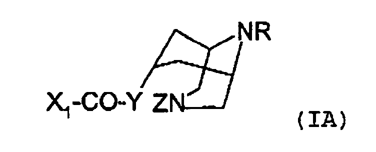 dans laquelle
Z est un groupe alkyle en C₁-C₆, un groupe phényle
ou un groupe alkyle en C₁-C₄ substitué par un groupe phényle dans lequel une chaîne phényle est facultativement substituée par un ou plusieurs groupes halo, alkoxy en C₁-C₆ ou alkyles en C₁-C₆;
R est un groupe méthyle ;
Y est NH ou O (ou est lié à R₁₀ tel que défini ci-dessous) ; et
X₁ est un groupe de formule (a), (b), (c), (d), (e), (f) ou (g) ; 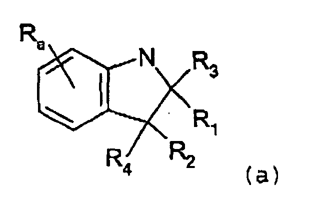 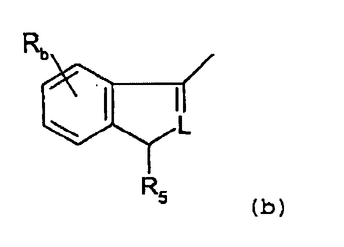 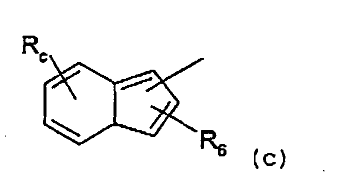 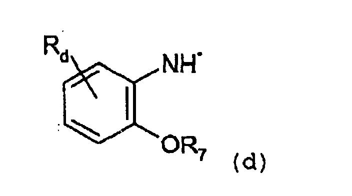 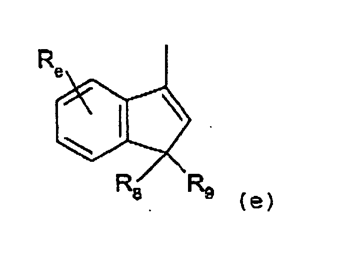 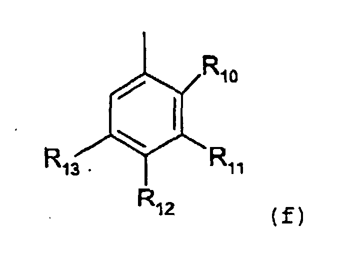 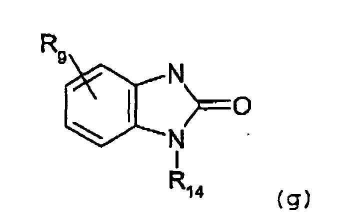 dans lesquelles
Rₐ à Rₑ et R_{g} sont choisis parmi l'hydrogène, les halogènes ou les groupes hydroxy ;
R₁ est un atome d'hydrogène et R₂ est un atome d'hydrogène ou un groupe alkyle en C₁-C₄ ; ou
R₁ et R₂ forment ensemble une liaison ;
R₃ à R₇ sont indépendamment un atome d'hydrogène ou un groupe alkyle en C₁-C₆ ; et
R₄ ensemble avec R₂ peut être un polyméthylène en C₂-C₇ ou un polyméthylène en C₂-C₆ interrompu par une liaison -O- lorsque R₁ est un atome d'hydrogène ;
R₈ et R₉ sont indépendamment choisis parmi l'hydrogène ou un groupe alkyle en C₁-C₆ ou R₈ et R₉ sont ensemble un polyméthylène en C₂-C₆ ou un polyméthylène en C₂-C₅ interrompu par une liaison -O- ;
soit R₁₀ est un atome d'hydrogène, un groupe alkoxy en C₁-C₆, un groupe cycloalkyloxy en C₃-C₈ ou un groupe alkyloxy en C₁-C₄ substitué par un groupe cycloalkyle en C₃-C₈ ; soit R₁₀ est lié à Y de sorte que Y-R₁₀ est N-B=N où B est N ou CH ; et
R₁₁ est un atome d'hydrogène, un groupe halo, un groupe alkoxy en C₁-C₆ ou un groupe alkyle en C₁-C₆ ; ou
R₁₀ et R₁₁ sont liés pour former -OCH (R₁₅R₁₆) -E- où E est (CH₂)ₙ ou NR₁₇CO(CH₂)ₘ où n est 1 ou 2 et m est 0 ou 1 et R₁₅, R₁₆ et R₁₇ sont indépendamment choisis parmi l'hydrogèn ou un groupe alkyle en C₁-C₆ ;
R₁₂ est un atome d'hydrogène ou un groupe alkoxy en C₁-C₆ ou un groupe amino facultativement substitué par un groupe alkyle en C₁-C₆ ou R₁₂ est un groupe alkamoylamino ; et
R₁₃ est un groupe halo, un groupe alkyle en C₁-C₆, un groupe alkoxy en C₁-C₆ ou un groupe alkylthio en C₁-C₆ ;
R₁₄ est un atome d'hydrogène ou un groupe alkyle en C₁-C₆ ; et
L est CH ou N ;
ledit procédé comprend
(i) la réaction d'un composé de formule (IV) :
X₁-COQ₁ (IV)
avec un composé de formule (V) : 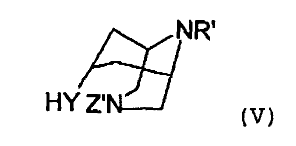 ou un dérivé réactif de celui-ci, lorsque Y est O ; dans lesquelles X₁' est X₁ ou un groupe convertible en celui-ci ; Q₁ est un groupe partant ; R' et Z' sont R et Z tels que définis ci-dessus ou un groupe de protection hydrogénolysable et les variables restantes sont telles que définies ci-dessus ; et ensuite facultativement la conversion de X₁' en X₁, y compris n'importe quel groupe Rₐ, R_{b}, R_{c}, R_{d}, Rₑ, R_{g} ou R₁₀, R₁₁, R₁₂, R₁₃ ou R₁₄ en un autre tel groupe ; la conversion de R'/Z', lorsque différent de R/Z, en R/Z ; et facultativement la formation d'un sel acceptable d'un point de vue pharmaceutique du composé résultant de formule (IA) ;
ou
(ii) l'N-alkylation d'un composé de formule (IA) sauf que R/Z est un atome d'hydrogène, l'N-alkylation comprenant la substitution des N-atomes azabicycliques par un groupe R/Z tel que défini ci-dessus et facultativement la formation d'un sel acceptable d'un point de vue pharmaceutique du composé résultant de formule (IA).

2. Procédé selon la revendication 1 dans lequel X présente la sous-formule (a), un de R₁ et R₃ est un atome d'hydrogène, R₂ est un groupe alkyle en C₁-C₄ et R₄ est un groupe alkyle en C₁-C₆ ou R₂ et R₄ sont liés pour former un polyméthylène en C₂-C₇.

3. Procédé selon la revendication 1 dans lequel X présente la sous-formule (b), et R₅ est un atome d'hydrogène ou un groupe méthyle ou éthyle.

4. Procédé selon la revendication 1 dans lequel X présente la sous-formule (d) et R₇ est un groupe méthyle.

5. Procédé selon la revendication 1 dans lequel X présente la sous-formule (f) dans laquelle R₁₀ est un groupe méthoxy, R₁₂ est un groupe amino et R₁₃ est un groupe chloro ou bromo.

6. Procédé selon l'une quelconque des revendications 1 à 5 dans lequel Z est un groupe méthyle.

7. Procédé selon la revendication 1 dans lequel X₁ est un groupe de formule (b) ; Y est NH ; R₅ est H, un groupe méthyle ou éthyle ; et Z est un groupe benzyle ou méthyle.

8. Procédé selon la revendication 7 dans lequel Z est un groupe méthyle.

9. Procédé selon la revendication 1 pour la préparation d'un composé de formule (I) qui est :
endo-4-amine-5-chloro-2-méthoxy-N-(3-benzyl-9-méthyl-3,9-diazabicyclo[3,3,1]nonan-7-yl)benzamide,
endo-4-amino-5-chloro-2-méthoxy-N-(3,9-diméthyl-3,9-diazabicyclo-[3,3,1]nonan-7-yl)benzamide,
endo-N-1-méthyl-3-indazolyl-(3-benzyl-9-méthyl-3,9-diazabicyclo[3,3,1]nonan-7-yl)carboxamide,
endo-N-3, 3-diméthylindolin-1-yl-(3-benzyl-9-méthyl-3,9-diazabicyclo[3,3,1]nonan-7-yl)carboxamide,
ou un sel acceptable d'un point de vue pharmaceutique d'un quelconque des composés mentionnés ci-dessus.

10. Procédé selon l'une quelconque des revendications 1 à 9 qui est le procédé (i) et dans lequel le groupe partant Q₁, déplaçable par un nucléophile, est un halogène.

11. Procédé selon l'une quelconque des revendications 1 à 9 qui est le procédé (i) et dans lequel le groupe partant Q₁, déplaçable par un nucléophile, est un groupe chloro ou bromo.

12. Procédé selon la revendication 10 ou 11 dans lequel la réaction s'effectue entre 0° et 100°C dans un solvant inerte non hydroxylique.

13. Procédé selon la revendication 12 dans lequel la réaction s'effectue en présence d'un accepteur d'acide.

14. Procédé selon l'une quelconque des revendications 1 à 9 qui est le procédé (i) et dans lequel le groupe partant Q₁, déplaçable par un nucléophile, est un groupe alkoxy en C₁-C₄, PhO-, Cl₅C₆O- ou Cl₃CO-.

15. Procédé selon la revendication 14 dans lequel la réaction est effectuée dans un solvant inerte polaire.

16. Procédé selon la revendication 15 dans lequel le solvant inerte polaire est le toluène ou le diméthylformamide.

17. Procédé selon l'une quelconque des revendications précédentes qui est le procédé (i) et dans lequel R'/Z' sont R/Z tels que définis dans les revendications 1 à 8 ou un groupe de protection hydrogénolysable qui est un groupe benzyle facultativement substitué par un ou deux groupes choisis parmi un groupe halo, un groupe alkoxy en C₁-C₄ ou un groupe alkyle en C₁-C₄.

18. Procédé selon l'une quelconque des revendications 1 à 9 qui est le procédé (ii) et dans lequel l'N-alkylation est obtenue par la réaction avec un composé RQ₃ ou ZQ₃ dans lequel R et Z sont définis dans les revendications 1 à 8 et Q₃ est un groupe partant déplaçable par des nucléophiles.

19. Procédé selon la revendication 18 dans lequel le groupe partant Q₃ est Cl, Br, I, OSO₂CH₃ ou OSO₂C₆H₄pCH₃.
